# EUROPEAN PATENT SPECIFICATION

(11) **EP 2 450 354 B1**
(45) Date of publication and mention of the grant of the patent: **17.08.2016**
(21) Application number: 10794264.1
(22) Date of filing: 05.07.2010
(51) Int. Cl.: C07D 249/08, A61K 51/00

(54) **LABELING COMPOUND FOR PET**
PET-KENNZEICHNUNGSKOMPONENTE
COMPOSÉ DE MARQUAGE POUR PET

(30) Priority: 03.07.2009 JP 2009159129
(43) Date of publication of application: 09.05.2012
(73) Proprietor: Riken, Wako-shi, Saitama 351-0198 (JP)
(72) Inventor: WATANABE, Yasuyoshi, Kobe-shi Hyogo 650-0047 (JP); ONOE, Hirotaka, Kobe-shi Hyogo 650-0047 (JP); TAKAHASHI, Kayo, Kobe-shi Hyogo 650-0047 (JP); SUZUKI, Masaaki, Kobe-shi Hyogo 650-0047 (JP); DOI, Hisashi, Kobe-shi Hyogo 650-0047 (JP); HOSOYA, Takamitsu, 136-0072 Tokyo (JP)
(74) Representative: Bittner, Thomas L.
(86) International application number: PCT/JP2010/061371
(87) International publication number: WO 2011/002096

(56) References cited:
- WO-A1-93/05027
- WO-A1-2008/023780
- JP-A- 2006 306 872
- JP-A- 2007 217 370
- US-A- 5 674 886
- MIN WANG ET AL: "Synthesis of a New Carbon-11-Labeled Sulfamate Derivative as a Potential PET Tracer for Imaging of Breast Cancer Aromatase and Steroid Sulfatase Expression", SYNTHETIC COMMUNICATIONS, vol. 41, no. 8, 25 June 2009 (2009-06-25), pages 1127-1140, XP55041815, ISSN: 0039-7911, DOI: 10.1080/00397911003797825
- OKADA MINORU ET AL: "Studies on aromatase inhibitors. II. Synthesis and biological evaluation of 1-amino-1H-1,2,4-triazole derivatives", CHEMICAL & PHARMACEUTICAL BULLETIN, PHARMACEUTICAL SOCIETY OF JAPAN, JP , vol. 45, no. 2 1 February 1997 (1997-02-01), pages 333-337, XP008157459, ISSN: 0009-2363 Retrieved from the Internet: URL:http://www.jstage.jst.go.jp/browse/cpb [retrieved on 2008-03-31]
- OKADA M ET AL: "STUDIES ON AROMATASE INHIBITORS. I. SYNTHESIS AND BIOLOGICAL EVALUATION OF 4-AMINO-4H-1,2,4-TRIAZOLE DERIVATIVES", CHEMICAL & PHARMACEUTICAL BULLETIN, PHARMACEUTICAL SOCIETY OF JAPAN, JP, vol. 44, no. 10, 1 October 1996 (1996-10-01), pages 1871-1879, XP000992853, ISSN: 0009-2363
- MASAFUMI KUDOH ET AL: "The potent and selective inhibition of estrogen production by non-steroidal aromatase inhibitor, YM511", THE JOURNAL OF STEROID BIOCHEMISTRY AND MOLECULAR BIOLOGY, vol. 54, no. 5-6, 1 September 1995 (1995-09-01), pages 265-271, XP55042212, ISSN: 0960-0760, DOI: 10.1016/0960-0760(95)00136-N
- WANG, M. ET AL.: 'Design and synthesis of carbon-11-labeled dual aromatase-steroid sulfatase inhibitors as new potential PET agents for imaging of aromatase and steroid sulfatase expression in breast cancer' STEROIDS vol. 74, 25 June 2009, pages 896 - 905, XP026587174
- AMETAMEY S M ET AL: "Molecular Imaging with PET", CHEMICAL REVIEWS, AMERICAN CHEMICAL SOCIETY, US, vol. 108, no. 5, 1 May 2008 (2008-05-01), pages 1501-1516, XP002724498, ISSN: 0009-2665, DOI: 10.1021/CR0782426 [retrieved on 2008-04-22]

## Description

### Technical Field

The present invention relates to a labeling compound for PET and to a diagnosis composition containing the compound.

### Background Art

Recently, various researches have been made in order to obtain in vivo information. In particular, a "molecular imaging" technique capable of analyzing the behavior of a molecule of interest attracts much attention. The molecular imaging is useful for finding of various diseases at their early stages and short-time development of effective medicine with little adverse effect.

A well known example of molecular imaging for obtaining in vivo information is Positron Emission Tomography (PET). PET is a technique which captures a tomographic image using gamma rays emitted when a positron collides with an electron. PET is carried out by labeling a molecule of interest with a positron-emitting radionuclide (so-called molecular probe), which is administered to a living body. The labeled radionuclide decays into another nucleus while emitting a positron, which collides with a neighboring electron to emit gamma rays being detected. This allows quantitative evaluation of the site where the molecule of interest exists. The PET is widely used as a clinical molecular imaging technique.

Aromatase which is known to be expressed in breast cancer etc. is an enzyme that converts male steroids into female steroids. Aromatase is also known to be related to mental disorders, menopausal syndrome, chronic fatigue syndrome etc. Therefore, it is considered that accurate quantification of aromatase is informative for diagnosing, determining the causes, and establishing therapeutic treatments for these diseases.

As a technique to image aromatase by PET, there is a reported technique using ¹¹C-labeled (S)-6-[(4-chlorophenyl) (1,2,4-triazol-1-yl)methyl]-1-[¹¹C]methylbenzotriazole([¹¹C]vorozole) as a labeling compound for PET (for example, see Non-patent Literatures 1 and 2).

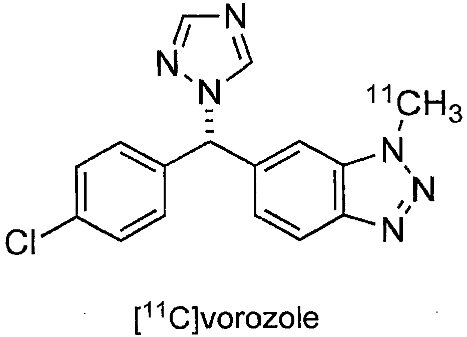

### Citation List

### [Non-patent Literatures]

[Non-patent Literature 1] Lidstrom,P.; Bonasera, T. A.; Kirilovas, D.; Lindblom, B.; Lu, L.; Bergstrom, E.; Bergstrom, M.; Westlin, J.-E.; Langstrom, B. Nucl. Med. Biol. 1998, 25, 497-501.
[Non-patent Literature 2] Takahashi, K.; Bergstrom, M.; Frandberg, P.; Vesstrom, E.-L.; Watanabe, Y.; Langstrom, B. Nucl. Med. Biol. 2006, 33, 599-605.

Wang et al. (Steroids 74, 2009, pp. 895-905) describes the design and synthesis of carbon-11-labeled dual aromatase-steroid-sulfatase inhibitors as potential PET agents for imaging of aromatase and steroid sulfatase expression in breast cancer. Compounds in accordance with the present invention are not disclosed.

Okada et al. ((a) Chem. Pharm. Bull., 1997, 45(2), pp. 333-337; (b) Chem. Pharm. Bull., 1996, 44(10), pp. 1871-1879) describe the synthesis and biological evaluation of 1-Amino-1H-1,2,4-triazole derivatives and 4-Amino-4H-1,2,4-triazole derivatives. Compounds in accordance with the present invention are not disclosed.

Kudoh et al. (J. Steroid Biochem. Molec. Biol., 1995, 54(5/6), pp. 265-271) describes compound YM511, a non-steroidal inhibitor of aromatase activity. Compounds in accordance with the present invention are not disclosed.

US 5,674,886 describes triazolylated tertiary amine compounds having aromatase inhibitory activity and being useful for preventing and treating breast cancer, mastopathy, endometriosis, and prostatomegaly. Compounds in accordance with the present invention are not disclosed.

Ametamey et al. (Chem. Ref. 2008, 108, pp. 1501-1516) is a review article on molecular imaging with PET. Compounds in accordance with the present invention are not disclosed.

### Summary of Invention

### Technical Problem

As described above, visualization of aromatase by PET imaging is actually used in practice. However, the compounds described in Non-patent Literatures 1 and 2 suffer a problem that N-¹¹CH₃ moiety in the probe may be cleaved metabolically and its metabolite may be taken into the brain again, which results in poor quantitative performance. Therefore, in order to quantify a molecule in a living body by PET, it is necessary to develop an excellent molecular probe.

The present invention was made in view of the foregoing problem, and the object is to provide a labeling compound for PET capable of quantifying aromatase with high accuracy.

### Solution to Problem

In order to solve the foregoing problem, a compound of the present invention is represented by any of the structures shown in claim 1 of the appended claims.

Also disclosed is a structure represented by formula (1) below 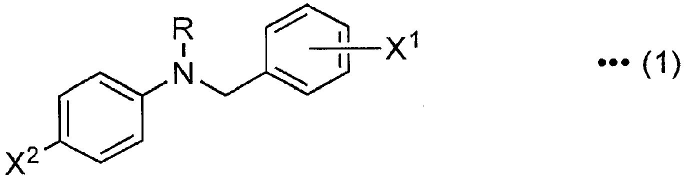 wherein X¹ is ¹¹CH₃, CH₂¹⁸F, CF₂¹⁸F, ¹⁸F, ⁷⁶Br or ¹²⁴I, X² is CN or NO₂, and R is any one of groups represented by 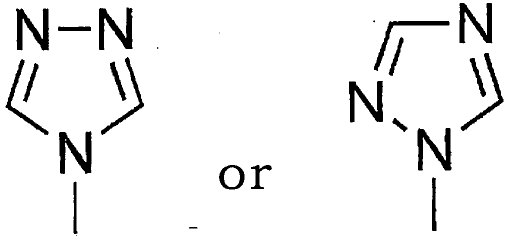 or a structure represented by formula (2) below 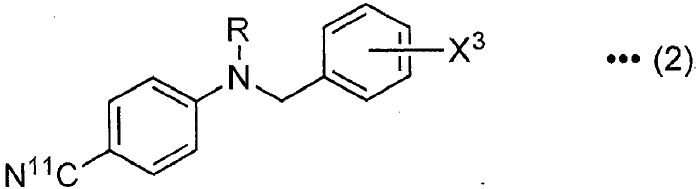 wherein X³ is CH₃, CH₂F, CF₃, F, Br or I, and R is defined equally as in the formula (1).

With the arrangement, the X¹ moiety and the ¹¹CN moiety are hardly to be cleaved metabolicallyThe compound of the present invention not only exhibits selective affinity toward aromatase but also allows quantifying, when used as a labeling compound for PET, aromatase with high accuracy. Consequently, the compound of the present invention facilitates diagnosis of various diseases, and is applicable to screening of candidate compounds for drugs targeting aromatase. Further, the compound of the present invention is applicable to, for example, screening of candidate compounds for drugs targeting molecules other than aromatase which molecules exhibit adverse effects by binding to aromatase.

Also disclosed is a structure represented by formula (1') 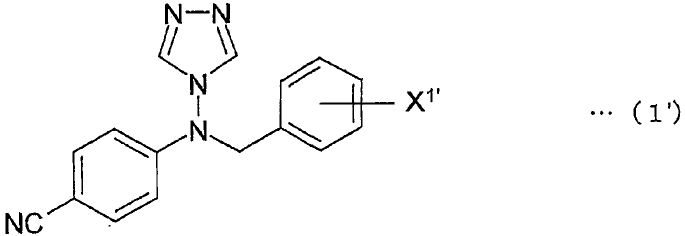 wherein X^{1'} is CH₃ or CH₂F.

The above compound has the same structure as that of the compound represented by general formula (1) or (2) except that the above compound is not radiolabeled. Accordingly, the above compound can be used as a control (so-called cold authentic) when the compound represented by general formula (1) or (2) is used as a labeling compound for PET.

A composition of the present invention for diagnosis includes the compound represented by any of the formulae shown in claim 1.

With the arrangement, the composition allows quantifying aromatase with high accuracy, and therefore allows diagnosing diseases suspected to be related to aromatase or sex steroids (e.g. breast cancer, mastopathy, endometrial cancer, endometriosis, uterine myoma) and diagnosing mental disorders, menopausal syndrome, and chronic fatigue syndrome etc. (including determining the causes thereof and establishing therapeutic treatments therefor). Further, the composition allows diagnosing inflammatory diseases with induction of aromatase. That is, the composition of the present invention for diagnosis is applied to diseases suspected to be related to aromatase or sex steroids (e.g. breast cancer, mastopathy, endometrial cancer, endometriosis, uterine myoma), inflammatory diseases with induction of aromatase, mental disorders, menopausal syndrome, and chronic fatigue syndrome. Further, the composition of the present invention for diagnosis is applicable to screening of candidate compounds for drugs targeting aromatase. Further, the compound of the present invention is applicable to, for example, screening of candidate compounds for drugs targeting molecules other than aromatase which molecules exhibit adverse effects by binding to aromatase.

Also disclosed is a method for diagnosis which includes the step of using, in PET, the compound of the present invention represented by any of the formulae of claim 1 or the composition of the present invention for diagnosis. The method for diagnosis can be a method for collecting data for assisting diagnosis.

Aromatase is an enzyme that converts male steroids to female steroids, and plays an important role in synthesizing sex steroids. By using the compound represented by general formula (1) or (2) as a labeling compound for PET, it is possible to collect, with high accuracy, data for assisting diagnosis of diseases suspected to be related to aromatase or sex steroids (e.g. breast cancer, mastopathy, endometrial cancer, endometriosis, uterine myoma). Further, it is possible to collect, with high accuracy, data useful for diagnosing mental disorders, menopausal syndrome, chronic fatigue syndrome etc.

Further, also disclosed is a compound which includes a structure represented by formula (3) 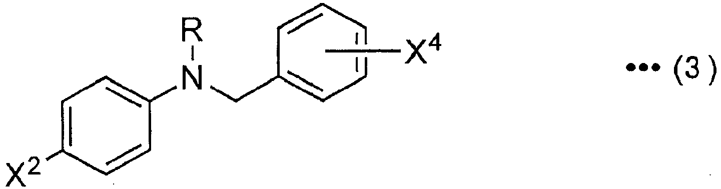 wherein X² is CN or NO₂, X⁴ is -CH₂X⁵, -CF₂X⁵, -SnR¹₃, -B(OH)₂, -BF₃·K, -B(OR²)(OR³) or 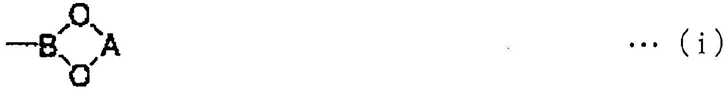 wherein A is a bivalent hydrocarbon group, and X⁵ is a leaving group selected from Cl, Br, I, -OSO₂CH₃, -OSO₂C₆H₄(4-CH₃) and -OSO₂CF₃, R¹, R², and R³ are independently an alkyl group having 1-6 carbon atoms, and R is any one of groups represented by 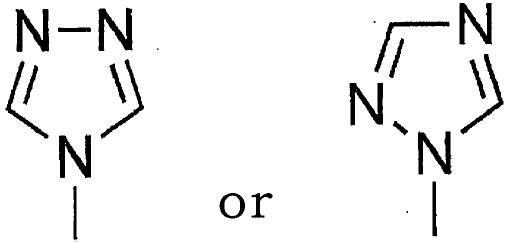 or a structure represented by formula (4) 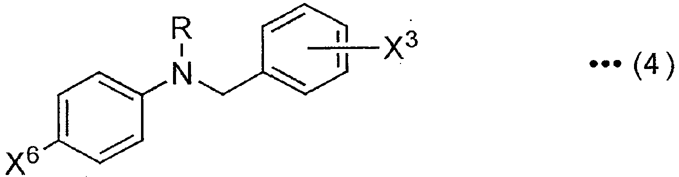 wherein X³ is CH₃, CH₂F, CF₃, F, Br or I, X⁶ is a leaving group selected from Cl, Br, I and -OSO₂CF₃, and R is defined equally as in the formula (3).

With the arrangement, the compound represented by formula (1) can be obtained by reacting, for example, the compound represented by formula (3) with labeled methyl iodide etc. or labeled potassium fluoride etc. or benzyl halide bearing a substituent X¹ on the benzene ring in advance etc. according to the method described in, for example, Non-patent Literature 3 (Okada, M.; Yoden, T.; Kawaminami, E.; Shimada, Y.; Kudoh, M.; Isomura, Y.; Shikama, H.; Fujikura, T. Chem. Pharm. Bull. 1996, 44, 1871-1879) or Non-patent Literature 4 (Okada, M.; Yoden, T.; Kawaminami, E.; Shimada, Y.; Kudoh, M.; Isomura, Y. Chem. Pharm. Bull. 1996, 45, 333-337).

Further, the compound represented by formula (2) can be obtained by reacting the compound represented by formula (4) with labeled hydrogen cyanide, labeled copper(I) cyanide etc.

Further, a kit for producing a labeling compound for PET includes the compound represented by any of the formulae 4, 12, 17 and 32 shown further below.

With the arrangement, it is possible to easily produce a labeling compound for PET.

### Advantageous Effects of Invention

As described above, the compound of the present invention includes a structure represented by any of the formulae shown in claim 1. Accordingly, the compound of the present invention can provide a labeling compound for PET capable of quantifying aromatase with higher accuracy, facilitating diagnosis of various diseases, and is applicable to screening of candidate compounds for drugs targeting aromatase. Further, the compound of the present invention is applicable to screening of candidate compounds for drugs targeting molecules other than aromatase which molecules exhibit adverse effects by binding to aromatase.

### Brief Description of Drawings

Fig. 1

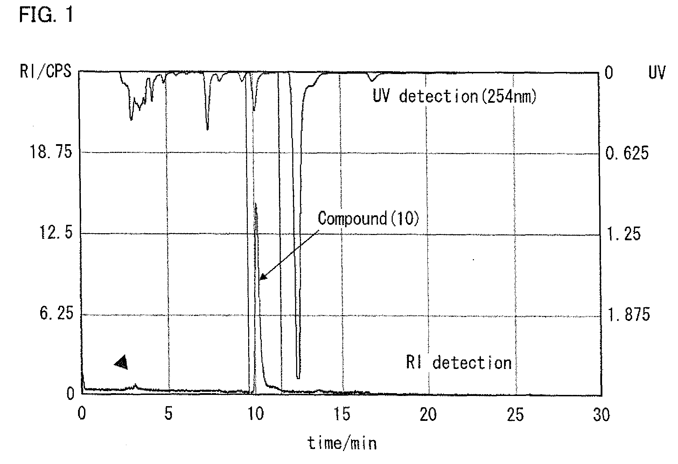

   Fig. 1 is a drawing showing the result of preparative HPLC of Compound (10) of the present invention.
Fig. 2

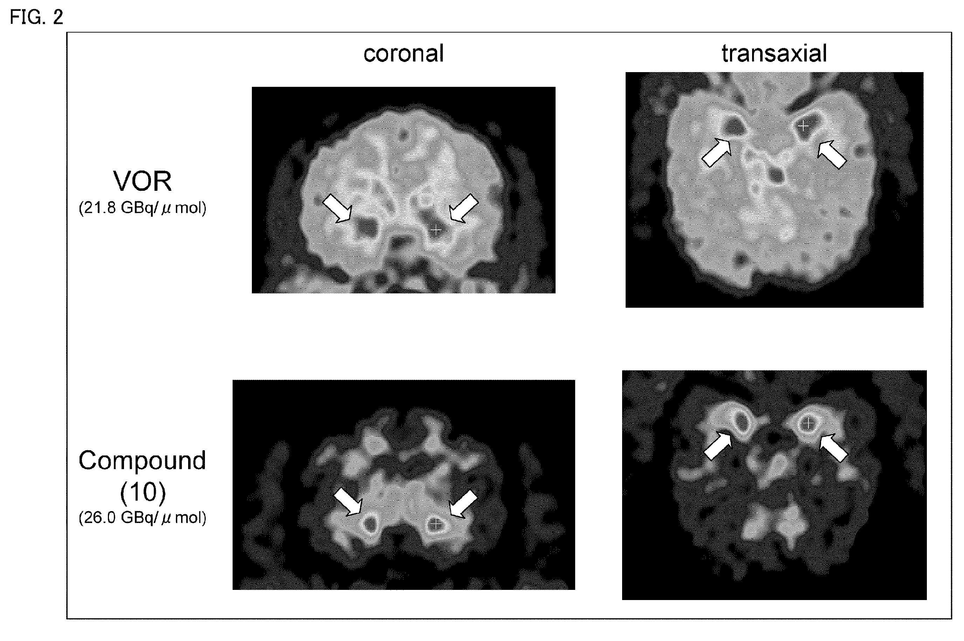

   Fig. 2 is a drawing showing binding potential images of amygdala in a case of using a labeling compound of the present invention and in a case of using a conventional labeling compound.
Fig. 3

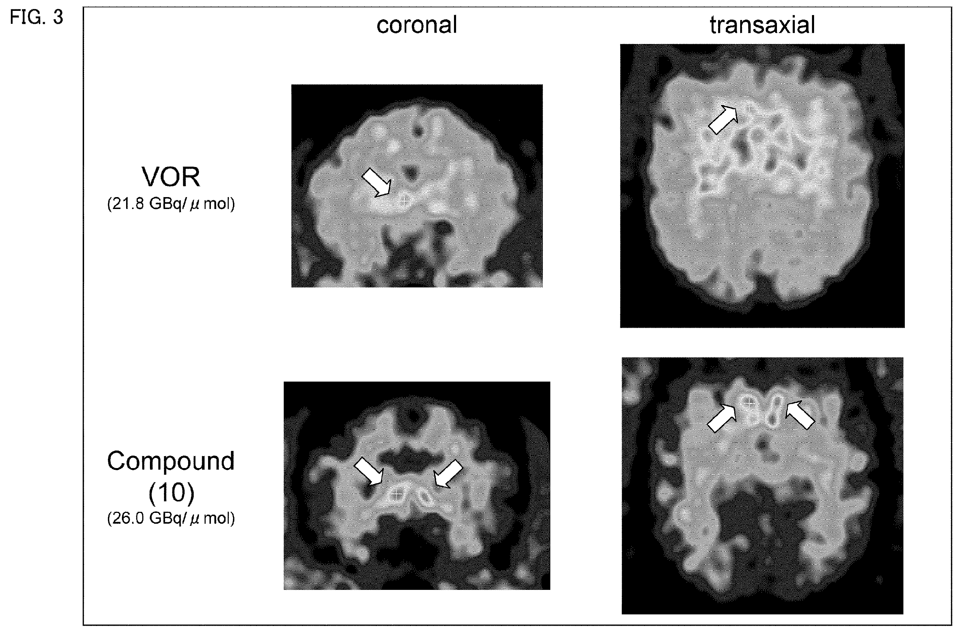

   Fig. 3 is a drawing showing binding potential images of nucleus accumbens in the case of using a labeling compound of the present invention and in the case of using a conventional labeling compound.
Fig. 4

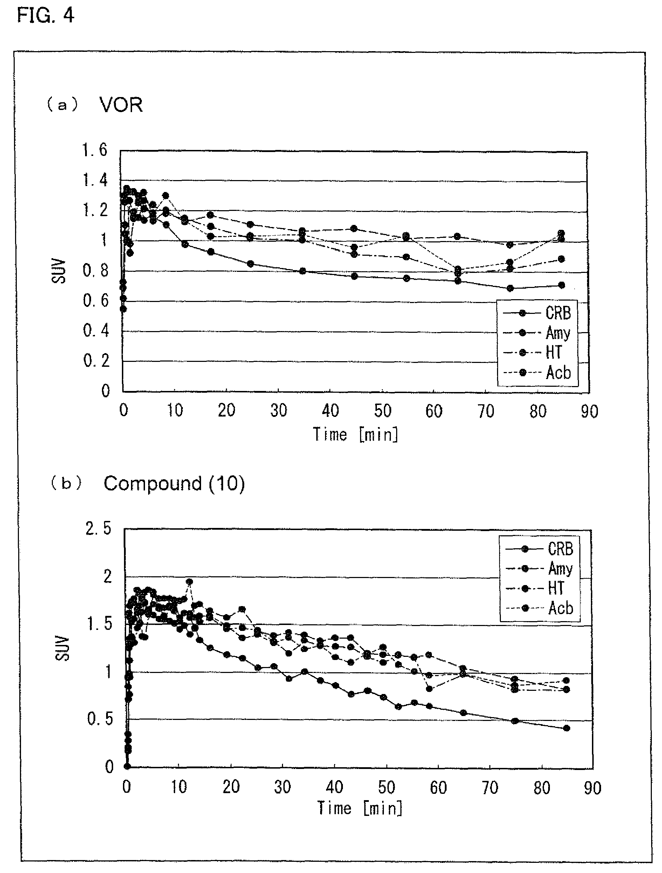

   Fig. 4 is a graph showing temporal changes in SUV in PET measurement of cerebellum, amygdala, hypothalamus and nucleus accumbens in the case of using a labeling compound of the present invention and in the case of using a conventional labeling compound.
Fig. 5

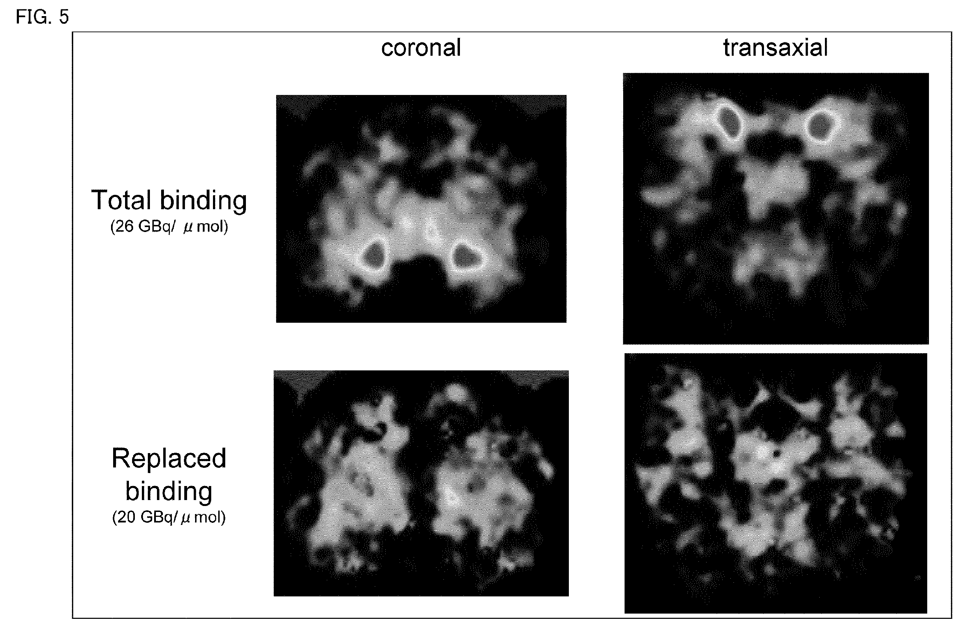

   Fig. 5 is a drawing showing binding potential images in a replacement test of Compound (10) of the present invention.
Fig. 6

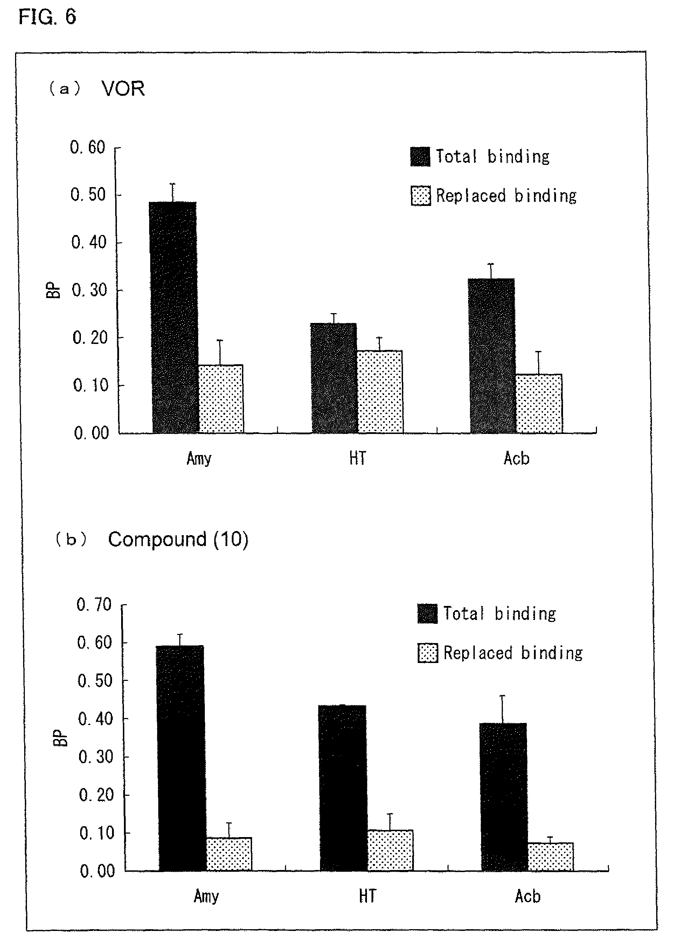

   Fig. 6 is a graph obtained by putting the results of the binding images in Fig. 5 in numerals.
Fig. 7

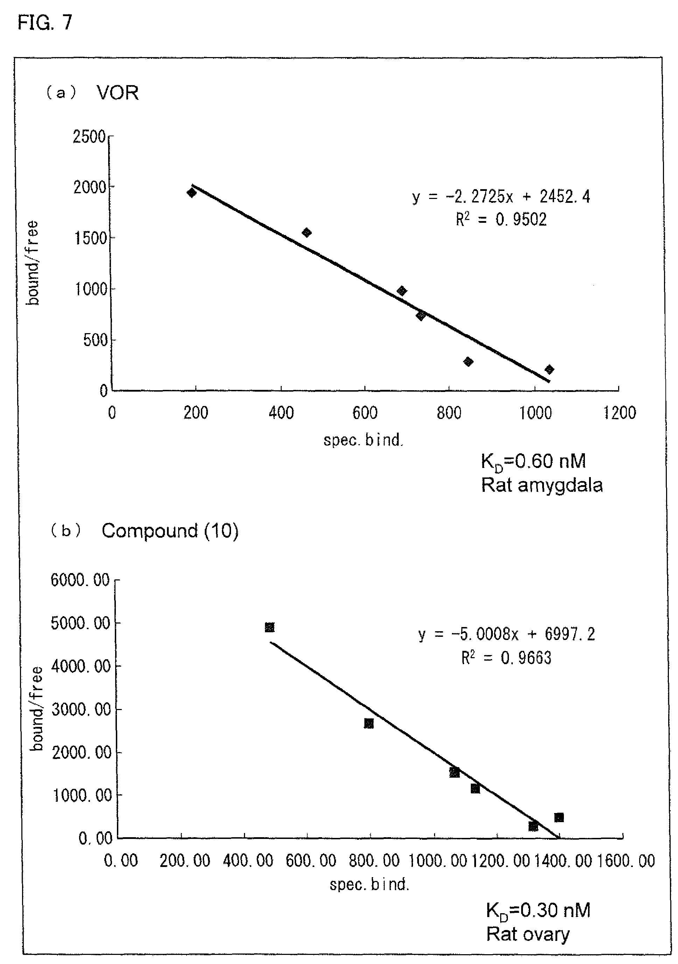

   Fig. 7 is a graph showing the result of a binding test of Compound (10) of the present invention.
Fig. 8

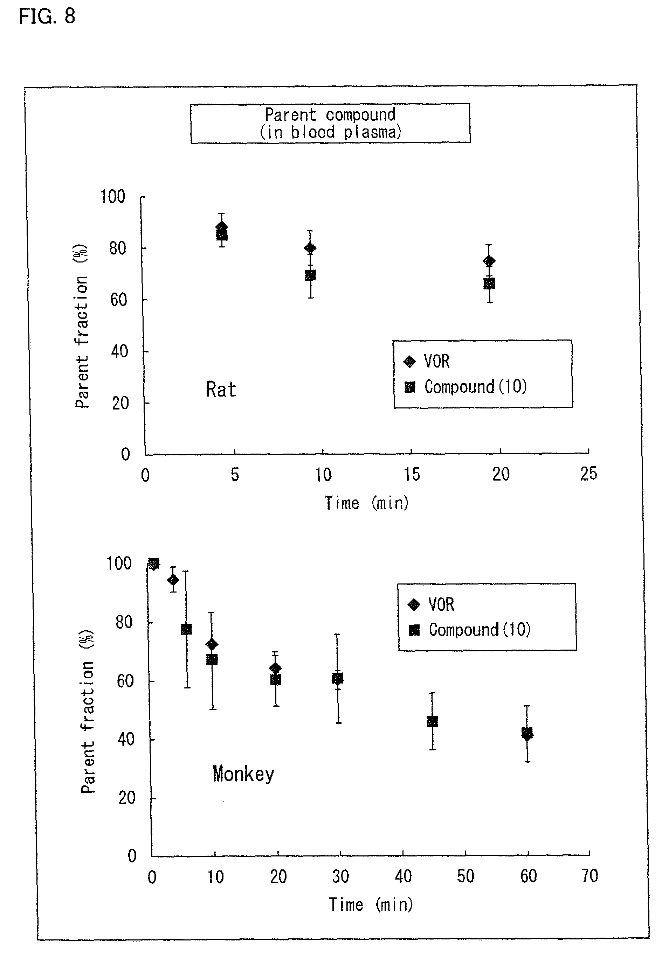

   Fig. 8 is a graph showing temporal changes in content of parent compound in blood in the case of using a labeling compound of the present invention and in the case of using a conventional labeling compound.
Fig. 9

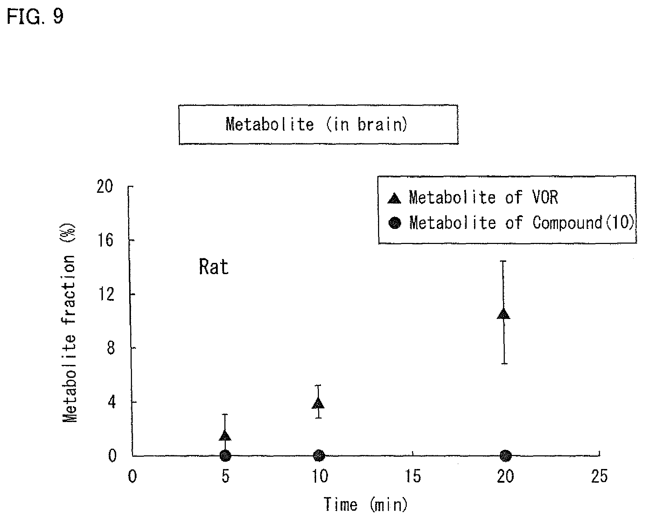

   Fig. 9 is a graph showing temporal changes in content of labeled metabolite in brain in the case of using a labeling compound of the present invention and in the case of using a conventional labeling compound.
Fig. 10

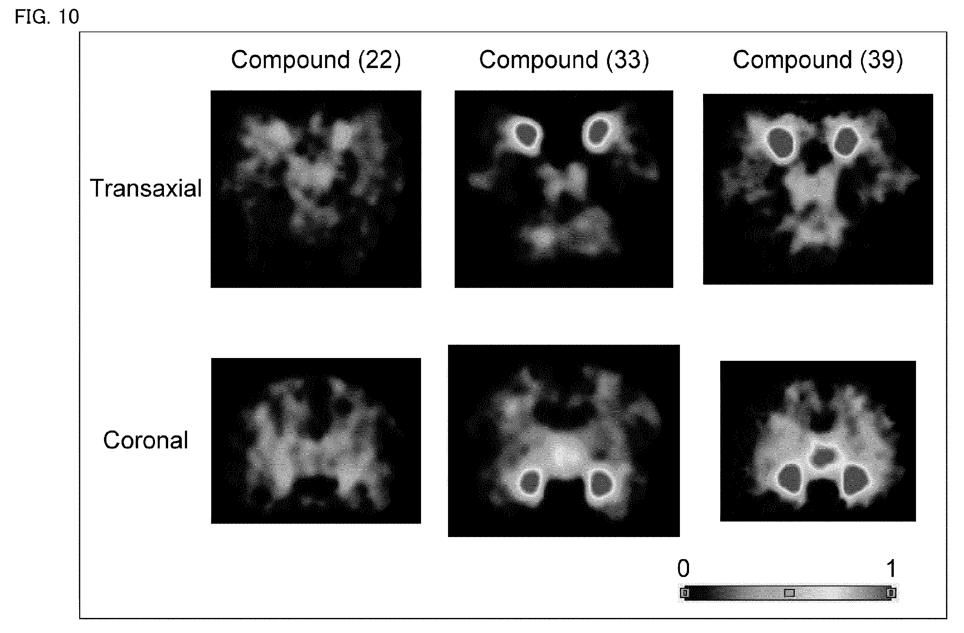

   Fig. 10 is a drawing showing binding potential images of amygdala in a case of using another labeling compounds of the present invention.
Fig. 11

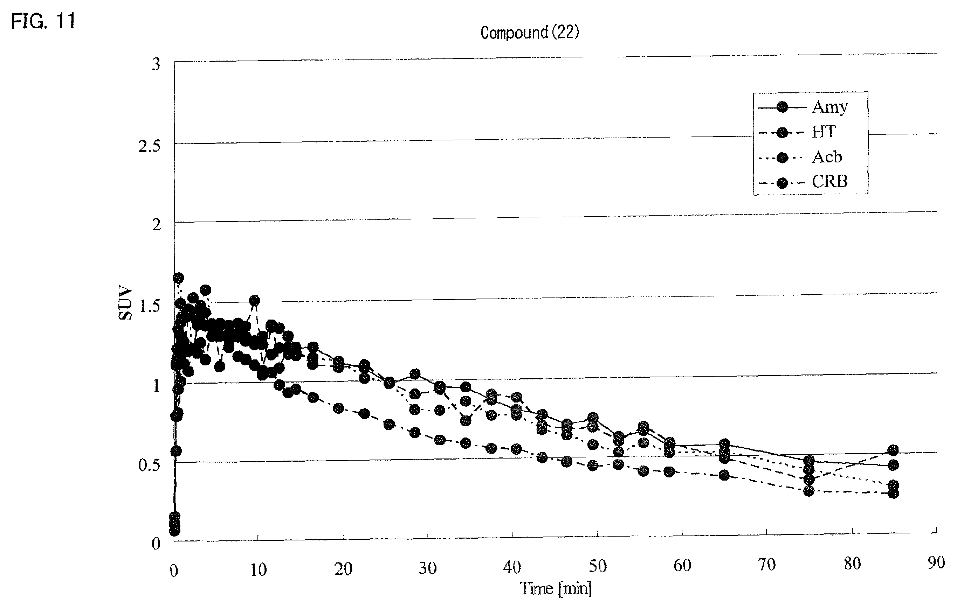

   Fig. 11 is a graph showing temporal changes in SUV in PET measurement of amygdala, hypothalamus, nucleus accumbens and cerebellum in a case of using Compound (22) of the present invention.
Fig. 12

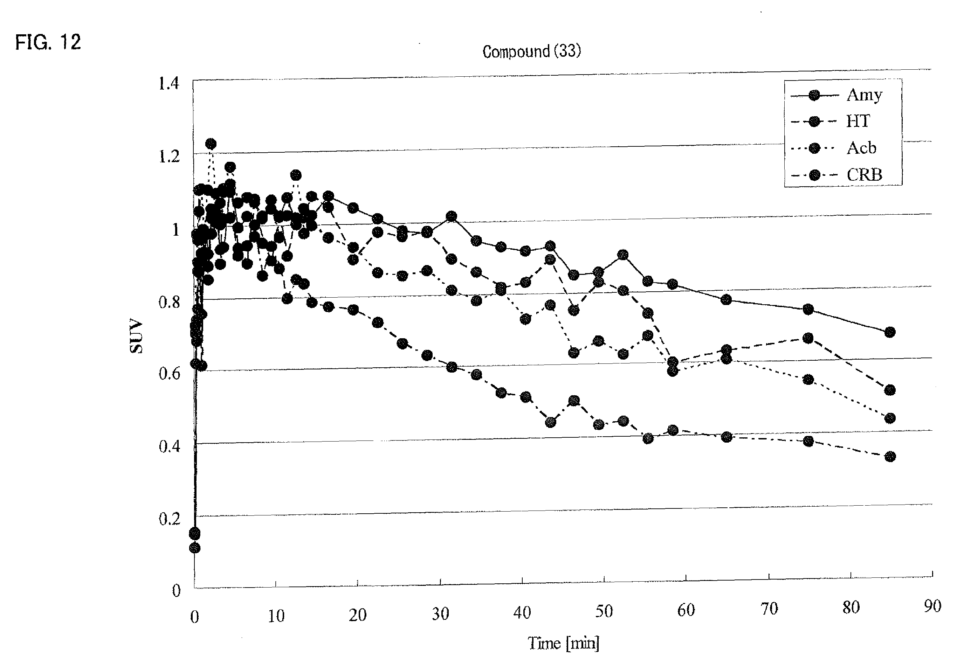

   Fig. 12 is a graph showing temporal changes in SUV in PET measurement of amygdala, hypothalamus, nucleus accumbens and cerebellum in a case of using Compound (33) of the present invention.
Fig. 13

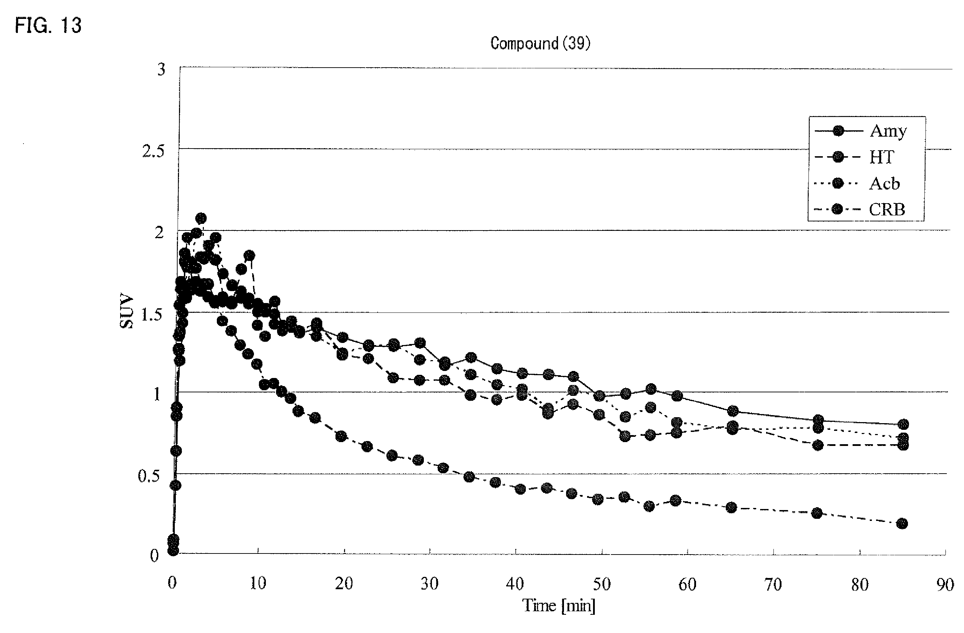

   Fig. 13 is a graph showing temporal changes in SUV in PET measurement of amygdala, hypothalamus, nucleus accumbens and cerebellum in a case of using Compound (39) of the present invention.

### Description of Embodiments

One embodiment of the present invention is explained below.

A compound in accordance with the present invention includes a structure represented by formulae (10), (22), (33), or (39) shown further below and in the appended claims.

Such a compound exhibits selective affinity toward aromatase which is an enzyme converting male steroids into female steroids. Further, the compound has a short-lived radionuclide in a metabolically stable moiety. Accordingly, the compound allows not only visualizing the distribution of aromatase but also providing a PET image with high quantitative performance.

¹¹CH₃ not only allows an examination with lower exposure of the subject to radiation but also allows carrying out consecutive examinations by other molecular probes for PET.

Also disclosed is a compound which has a structure represented by formula (1") below 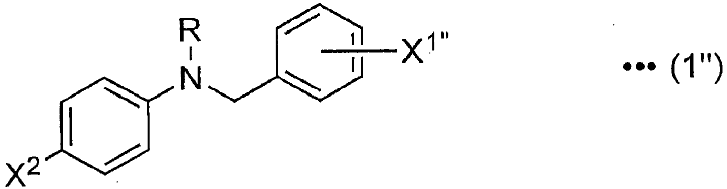 wherein X^{1"} is CH₃, CH₂F, CF₃, F, Br or I, X² is CN or NO₂, and R is defined equally as in the formula (1).

The above compound has the same structure as that of the compound represented by general formula (1) or general formula (2) or by any of the formulae (10), (22), (33), or (39) except that the above compound is not radiolabeled. Accordingly, the above compound can be preferably used as a cold authentic in a case where the compound represented by general formula (1) or general formula (2) or formulae (10), (22), (33), or (39) is used as a labeling compound for PET.

The compound represented by formula (1") preferably has a structure represented by formula (1') below 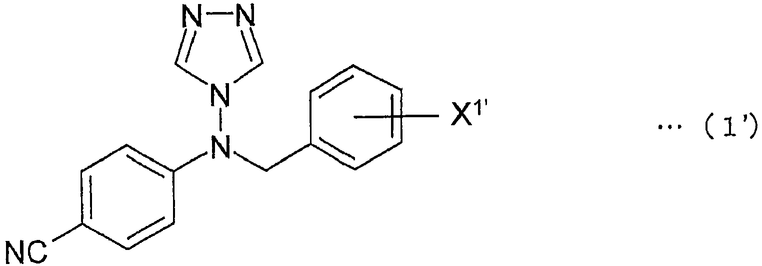 wherein X^{1'} is CH₃ or CH₂F.

Further, X^{1'} in formula (1') above is more preferably CH₃.

Substituted position of X^{1"} in formula (1") and substituted position of X^{1'} in formula (1') may be the same as those of X¹ or X³ in the compound represented by formula (1) or formula (2), and may be any one of ortho-position, meta-position, and para-position.

The compound represented by formula (1) can be synthesized from a compound having a structure represented by formula (3) below 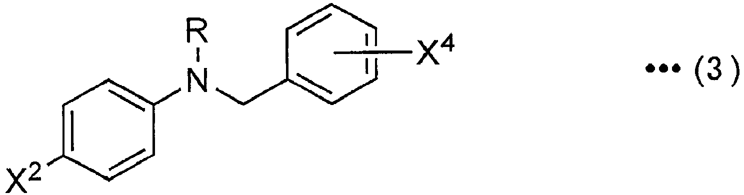 wherein X² is CN or NO₂, X⁴ is -CH₂X⁵, -CF₂X⁵, -SnR¹₃, -B(OH)₂, -BF₃·K, -B(OR²)(OR³) or 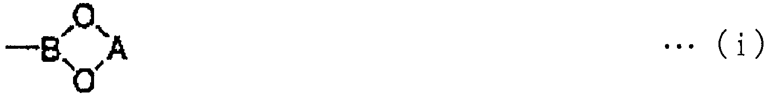 wherein A is a bivalent hydrocarbon group, and X⁵ is a leaving group such as Cl, Br, I, -OSO₂CH₃, -OSO₂C₆H₄(4-CH₃) and -OSO₂CF₃ etc., R¹, R², and R³ are independently an alkyl group having 1-6 carbon atoms, and R is defined equally as in general formula (1).

Specifically, in a case where X⁴ is -SnR¹₃, -B(OH)₂, -BF₃·K, -B(OR²)(OR³) or a substituent represented by formula (i), reacting the compound represented by formula (3) with labeled methyl iodide (¹¹CH₃I) or labeled fluoromethyl iodide (¹⁸FCH₂I) (which may be hereinafter generally referred to as "labeled methyl iodide etc.) allows obtaining the compound represented by formula (1) wherein X¹ is ¹¹CH³ or CH₂¹⁸F.

The above reaction in the case where X⁴ is -SnR¹₃ may be performed similarly with the method of reacting an organic tin compound with methyl iodide in the presence of palladium complex to introduce a methyl group as described in, for example, Non-patent Literature 5 (Suzuki, M.; Doi, H.; Bjorkman, M.; Andersson, Y.; Langstrom, B.; Watanabe, Y.; Noyori, R. Chem. Eur. J. 1997, 12, 2039-2042). Specifically, for example, the compound represented by formula (1) can be obtained by reacting, for a short time, the compound represented by formula (3) with labeled methyl iodide etc. in the presence of palladium(0) complex, ligand or carbonate and optionally copper halide or alkaline metal halide.

[¹¹C]Methylation and [¹⁸F]fluoromethylation from boron compounds may be performed similarly with the methods described in, for example, Non-patent Literature 6 (Doi, H.; Ban, I.; Nonoyama, A.; Sumi, K.; Kuang, C.; Hosoya, T.; Tsukada, H.; Suzuki, M. Chem. Eur. J. 2009,15,4165-4171) and International Publication WO2008/023780.

Further, in the case where X⁴ is -CH₂X⁵, the compound represented by formula (1) wherein X¹ is CH₂¹⁸F can be obtained by reacting the compound represented by formula (3) with labeled potassium fluoride (K¹⁸F) etc.

The compound represented by formula (1) wherein X¹ is CF₂¹⁸F can be obtained by nucleophilic substitution of the compound represented by formula (3) wherein X⁴ is -CF₂Br or -CF₂Cl using [¹⁸F]fluoride anion labeled with ¹⁸F similarly with the methods described in, for example, Non-patent Literature 7 (Prabhakaran, J.; Underwood, M. D,; Parsey, R. V.; Arango, V.; Majo, V. J.; Simpson, N. R.; Heertum, R. V.; Mann, J. J.; Kumar, J. S. D. Bioorg. Med. Chem. 2007, 15, 1802-1807) and Non-patent Literature 8 (Angelini, G.; Speranza, M.; Shiue, C.-Y.; Wolf, A. P. J. Chem. Soc.; Chem. Commun. 1986, 924-925).

The compound represented by formula (1) wherein X¹ is ¹⁸F can be obtained by reacting the compound represented by formula (3) wherein X⁴ is -SnR¹₃ with [¹⁸F]fluorine gas, [¹⁸F]-acetyl hypofluorite etc. which are labeled with ¹⁸F.

The compound represented by formula (1) wherein X¹ is ⁷⁶Br or ¹²⁴I can be obtained by reacting the compound represented by formula (3) wherein X⁴ is -SnR¹₃ with [⁷⁶Br]bromide anion labeled with ⁷⁶Br or [¹²⁴I]iodine anion labeled with ¹²⁴I in the presence of an oxidant such as chloramine T.

It is preferable that the A is a bivalent hydrocarbon group and is one of groups represented by the formula below.

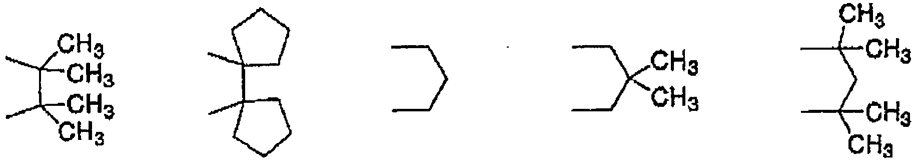

In a case where X⁴ is -CF₂X⁵ in formula (3), it is preferable that X⁵ is Cl or Br.

R¹, R², and R³ are alkyl groups having 1-6 carbon atoms, and preferably alkyl groups having 4-6 carbon atoms. Specific examples of R¹ include methyl group, ethyl group, n-propyl group, i-propyl group, cyclopropyl group, n-butyl group, i-butyl group, sec-butyl group, tert-butyl group, cyclopropylmethyl group, cyclobutyl group, n-pentyl group, i-pentyl group, sec-pentyl group, tert-pentyl group, 2-methylbutyl group, cyclobutylmethyl group, cyclopentyl group, n-hexyl group, 1-methylpentyl group, 2-methylpenthyl group, 3-methylpentyl group, 4-methylpentyl group, 1-ethylbutyl group, 2-ethylbutyl group, 1,1-dimethylbutyl group, 2,2-dimethylbutyl group, 3,3-dimethylbutyl group, 1-ethyl-1-methylpropyl group, cyclopentylmethyl group, and cyclohexyl group.

The palladium(0) complex is not particularly limited as long as it catalyzes a coupling reaction between the compound represented by formula (1) and labeled methyl iodide etc. Examples of the palladium(0) complex include Pd₂(dba)₃, Pd₂(dba)₃CHCl₃, Pd[P(o-CH₃C₆H₄)₃]₂, and Pd[P(tert-C₄H₉)₃]₂.

The ligand is not particularly limited. In the case where the palladium(0) complex is Pd₂(dba)₃, an example of the ligand is P(o-CH₃C₆H₄)₃.

Examples of the copper halide include CuCl and CuBr. For the amount of copper halide, for example, two or more equivalents for palladium atoms included in the palladium(0) complex can be used.

An example of the alkaline metal halide is CsF. For the amount of the alkaline metal halide, for example, two or more equivalents for palladium atoms included in the palladium(0) complex can be used.

Examples of the carbonate include potassium carbonate and cesium carbonate. The carbonate is preferably potassium carbonate. For the amount of the carbonate used, for example, two or more equivalents for palladium atoms included in the palladium(0) complex can be used.

A solvent may be used in the above reaction for obtaining the compound represented by formula (1). Examples of the solvent include DMF and a mixture of DMF and water.

The compound represented by formula (2) can be obtained by reacting the compound having a structure represented by formula (4) below 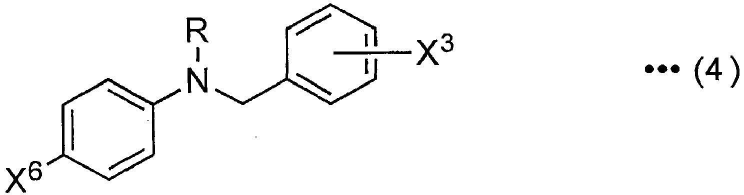 wherein X³ is defined equally as in general formula (2), X⁶ is a leaving group such as Cl, Br, I or OSO₂CF₃, and R is defined equally as in the formula (1), with labeled hydrogen cyanide (H¹¹CN), labeled copper(I) cyanide (Cu¹¹CN) or etc.

More specifically, in the case of reacting the compound having a structure represented by formula (4) with labeled copper(I) cyanide, the compound represented by formula (2) can be obtained by heating the reaction mixture at approximately at 180 °C in the presence of DMF. In the case of reacting the compound having a structure represented by formula (4) with labeled hydrogen cyanide, the compound represented by formula (2) can be obtained by heating the reaction mixture in the presence of a palladium catalyst such as (Ph₃P)₄Pd.

The substituted position of X³ in formula (4) may be the same as that of X³ in the compound represented by formula (2), and may be any of ortho-position, meta- position, and para- position.

The compound represented by formula (3) can be prepared from a compound having a structure represented by formula (5) below 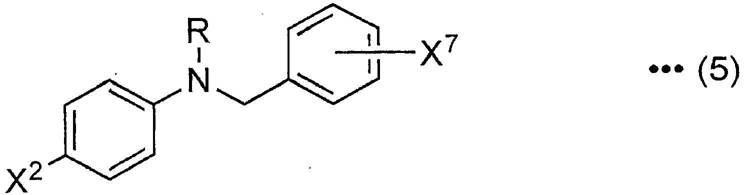 wherein X² is defined equally as in formula (3), X⁷ is a leaving group such as Cl, Br, I or OSO₂CF₃, and R is defined equally as in the formula (1).

Substituted position of X⁷ in formula (5) may be the same as that of X⁴ in the compound represented by formula (3), and may be any of ortho-position, meta- position, and para- position.

In the case where X⁴ in formula (3) is SnR¹₃, the compound represented by formula (3) can be prepared by reacting the compound represented by formula (5) with (SnR¹₃)₂ in the presence of Pd(PPh₃)₄.

In the case where X⁴ in formula (3) is a group represented by -B(OR²)(OR³) or formula (i), the compound represented by formula (3) can be prepared by reacting the compound represented by formula (3) with alkoxydiboron, which can supply the group represented by formula (i), in the presence of PdCl₂(dppf) and potassium acetate, according to, for example, Non-patent Literature 9 (Ishiyama, T.; Murata, M.; Miyaura, N. J. Org. Chem. 1995, 60, 7508-7510).

In the case where X⁴ in formula (3) is -B(OH)₂ or -BF₃·K, the compound represented by formula (3) can be prepared from pinacol boronic acid ester by the method described in Non-patent Literature 10 (Murphy, J. M.; Tzschucke, C. C.; Hartwig, J. F. Org. Lett. 2007, 9, 757-760).

In the case where X⁴ in formula (3) is CH₂X⁵, the compound represented by formula (3) can be prepared by reacting the compound represented by formula (5) wherein X⁵ is OH (hydroxyl group) with appropriate sulfonyl chloride or phosphorus trihalide in the presence of a base such as triethylamine.

In the case where X⁴ in formula (3) is CF₂X⁵, the compound represented by formula (3) wherein X⁴ is CF₂X⁵ can be prepared by reacting the compound represented by formula (3) wherein X⁵ is H (hydrogen) with chlorine, bromine, or N-bromosuccinimide. The same is applied to the case where X⁵ is I, -OSO₂CH₃, or -OSO₂CF₃.

The compound represented by formula (5) can be synthesized using benzyl halide etc. bearing a substituent X⁷ on a benzene ring in advance by a method described in, for example, Non-patent Literature 3 (Okada, M.; Yoden, T.; Kawaminami, E.; Shimada, Y.; Kudoh, M.; Isomura, Y.; Shikama, H.; Fujikura, T. Chem. Pharm. Bull. 1996, 44, 1871-1879) or Non-patent Literature 4 (Okada, M.; Yoden, T.; Kawaminami, E.; Shimada, Y.; Kudoh, M.; Isomura, Y. Chem. Pharm. Bull. 1996, 45, 333-337). The compound represented by formula (4) can be synthesized similarly.

The compound represented by formula (1") can be obtained by reacting 4-(4H-1,2,4-triazole-4-ylamino)benzonitrile (see Compound (3) in the Examples), 1-nitro-4-(4H-1,2,4-triazole-4-ilamino)benzene, 4-(1H-1,2,4-triazole-1-ylamino)benzonitrile or 1-nitro-4-(1H-1,2,4-triazole-1-ylamino)benzene with methylbenzyl halide, fluoromethylbenzyl halide, trifluoromethylbenzyl halide, fluorobenzyl halide, chlorobenzyl halide, bromobenzyl halide, iodobenzyl halide etc. in the presence of a base such as potassium carbonate. Further, the compound represented by formula (1) or (2) can be similarly prepared also by using unlabeled methyl iodide etc. (CH₃I, FCH₂I) instead of labeled methyl iodide etc., or using unlabeled hydrogen cyanide or copper(I) cyanide instead of labeled hydrogen cyanide or labeled copper(I) cyanide etc.

In principle, the compounds represented by formulae (1) and (2) can be obtained also by using benzyl halides labeled with positron emitting nuclide or benzonitriles labeled with positron emitting nuclide in the method described in Non-patent Literature 3 or 4.

Preventing the decrease in radioactivity of the molecular probe allows PET with smaller amount of administration. The inventors of the present invention have so far developed the methylation reaction which completes in a very short time. This methylation reaction allows using efficiently, as a molecular probe for PET, the compound prepared using a radionuclide with a very short lifetime. That is, in a preferred embodiment of the present invention, the compound represented by formula (1) may be a one to which the methylation reaction has been applied. Further, it is desirable that the compound is prepared by the methylation reaction.

In a case where the kit includes the compound represented by formula (3), the kit may further include at least one selected from the group consisting of labeled methyl iodide etc., palladium(0) complex, ligand, and carbonate. Optionally, the kit may further include copper halide or alkaline metal halide, other reagent used in the above reaction, solvent, and catalyst.

In a case where the kit includes the compound represented by formula (4), the kit may further include the labeled hydrogen cyanide (H¹¹CN) or the labeled copper(I) cyanide (Cu¹¹CN). Optionally, the kit may further include a solvent and a catalyst used in a reaction.

The term "kit" used herein indicates a package including a container (e.g. bottle, plate, tube, and dish) containing therein a specific material. The kit preferably has directions for using individual materials. The term "includes (including)" used in the aspect of the kit in the present specification indicates that a material is contained in any one of individual containers constituting the kit. Further, the kit of the present invention may be a package in which a plurality of different compositions are contained. The "directions" may be written or printed on a paper or other kind of a medium. Alternatively, the "directions" may be in the form of a magnetic tape or an electronic medium such as a computer-readable disc or tape and a CD-ROM.

Further, the kit may include a container containing a diluent, a solvent, a cleaning liquid, or other reagent. Further, the kit may include an instrument necessary for application.

It is known that aromatase appears in breast cancer, mastopathy, endometrial cancer, endometriosis, uterine myoma etc. Accordingly, PET with the compound represented by formula (1) or (2) as a labeled compound helps diagnosing these diseases, collecting data for helping diagnosis of these diseases, and developing therapeutic drugs (aromatase inhibitors). That is, also disclosed is a method for diagnosing a disease, a method for collecting data for helping diagnosing a disease, and a method for screening a candidate compound for a drug targeting aromatase or a candidate compound for a drug targeting molecules other than aromatase which exhibit adverse effect by binding to aromatase, each method including the step of using the compound represented by formula (1) or (2) in PET.

Further, it is known that aromatase is related to mental disorders, menopausal syndrome, chronic fatigue syndrome etc. Accordingly, quantifying the amount of aromatase in the brain using the compound represented by formula (1) or (2) as a labeling compound for PET contributes to determining the causes and establishing therapeutic treatments for these diseases.

In one embodiment, the compound of the present invention may be provided in the form of a composition. The composition in accordance with the embodiment may be used not only for imaging aromatase by PET but also for diagnosing diseases suspected to be related to aromatase or sex steroid (e.g. breast cancer, mastopathy, endometrial cancer, endometriosis, uterine myoma) or for diagnosing mental disorders, menopausal syndrome, chronic fatigue syndrome etc. The composition in accordance with the embodiment may be prepared in the form of a solution or a suspension or in the form of a solid suitable for being dissolved or suspended in a liquid (e.g. buffer).

Further, the composition in accordance with the present embodiment may further contain the compound represented by formula (1").

The composition in accordance with the present embodiment may be provided in the form of a kit. The term "composition" indicates a substance in which two or more components are contained. The term "kit" indicates a substance in which at least one of the components is contained in other material. In the specification, a "composition containing a main component" encompasses a "kit containing a main component". Other components contained in the composition are not particularly limited as long as the components do not hamper the function of the compound which is a main component.

It is desirable that administration of the compound in accordance with the present embodiment to a living body may be performed by injection via various routes, but is not limited to it. The amount of administration may be appropriately determined by a person skilled in the art according to necessity.

As described above, the compound of the present invention is a compound represented by any of formulae (10), (22), (33) or (39) all of which comprise ¹¹CH₃.

With the arrangement, since ¹¹CH₃ has a relatively short half-life, it is possible to reduce the exposure of the subjects to radiation and thus to carry out consecutive examinations using other labeling compounds for PET.

Also disclosed is a compound represented by formula (2) wherein X³ is preferably CH₃ or Br.

Further, it is preferable that the composition of the present invention for diagnosis further includes a compound having a structure represented by formula (1") below 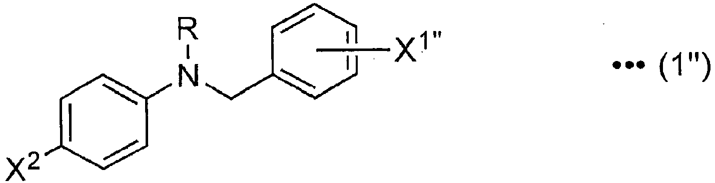 wherein X¹" is CH₃, X² is CN or NO₂, and R is any one of groups represented by formulae below wherein said compound of formula (1") has the same structure as that of the compounds of claim 1, except that the compound of formula (1") is not radiolabelled.

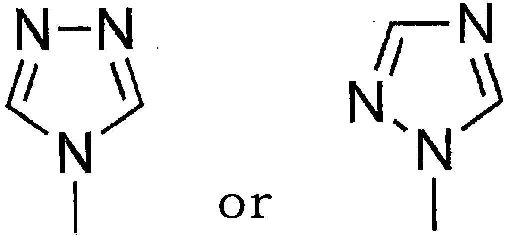

Further, the composition of the present invention for diagnosis is preferably applied to a disease selected from the group consisting of diseases suspected to be related to aromatase or sex steroids, inflammatory diseases with induction of aromatase, mental disorders, menopausal syndrome, and chronic fatigue syndrome.

Further, the method for diagnosis is preferably applied to a disease selected from the group consisting of diseases suspected to be related to aromatase or sex steroids, inflammatory diseases with induction of aromatase, mental disorders, menopausal syndrome, and chronic fatigue syndrome.

### [Examples]

The following explains the present invention in more details by referring to Examples. It should be noted that the present invention is not limited to the Examples.

In the experiment below, TLC (thin-layer chromatography) was carried out using a plate precoated with silica gel of 0.25 mm in thickness (Merck Chemicals, Silica Gel 60 F254, Cat. No. 1.05715).

Column chromatography was carried out using silica gel (KANTO CHEMICAL CO., INC., Silica Gel 60N, spherical neutral, particle diameter 40-50 µm, Cat. No. 37563-85 or particle diameter 63-210 µm, Cat. No. 37565-85)

The melting point (Mp) was measured by YANACO MP-J3 instrument, and was not corrected.

¹H and ¹³C NMR were measured by Varian MERCURY 300 spectrometer at 300 MHz and 75.5 MHz, respectively. Alternatively, ¹H and ¹³C NMR were measured by JEOL JNM-EX270 spectrometer at 270 MHz and 67.8 MHz, respectively. Solutions for NMR spectrum were CDCl₃ (CIL, Cat. No. DLM-7TB) and DMSO-d₆ (CIL, Cat. No. DLM-10). Chemical shift (δ) is expressed in ppm with a coupling constant (Hz) using (CH₃)₄Si (δ 0.00 (¹H NMR) in case of CDCl₃) or the peak of the above solvent (δ 2.49 (¹H NMR) and δ 39.5 (¹³C NMR) in case of DMSO-d₆, and δ 77.0 (¹³C NMR) in case of CDCl₃) as an internal reference. Abbreviations "s", "d", "t", "m", and "br" represent "singlet", "doublet", "triplet", "multiplet" and "broad", respectively.

IR spectrum was measured by a diffuse reflection method using SHIMADZU IR Prestige-21 spectrometer equipped with a DRS-8000A unit.

Elemental analysis was made using YANACO CHN CORDER MT-5.

High resolution mass spectrum (HRMS) was measured by JEOL JMS-700 mass spectrometer under the condition of positive fast atom bombardment (FAB+). Alternatively, HRMS was measured by Bruker micrOTOF mass spectrometer under the condition of positive electrospray ionization (ESI+).

In the present Examples, Compound (1) (4-fluorobenzonitrile) (Cat. No. 329-70013), Compound (2) (4-amino-4H-1,2,4-triazole) (Cat. No. 326-41573), potassium tert-butoxide (Cat. No. 161-08421), 4-methylbenzyl bromide (Cat. No. 021-03131), 4-bromobenzyl bromide (Cat. No. 325-36072), potassium carbonate (Cat. No. 162-03495), Compound (5) (4-bromobenzyl alcohol) (Cat. No. 320-77123), tributyltin(IV) chloride (Cat. No. 202-08981), methanesulfonyl chloride (Cat. No. 131-01583), triethylamine (Cat. No. 202-02646), bis(pinacolato)diboron (Cat. No. 329-56970), m-bromobenzyl bromide (Cat. No. 32-6642), p-fluoronitrobenzene (Cat. No. 064-02081), Compound (18) (3-bromobenzyl alcohol) (Cat. No. 326-21852), sodium bromide (Cat. No. 193-01505), Compound (26) (p-xylene-α, α'-diol) (Cat. No. 248-00562), and carbon tetrabromide (Cat. No. 038-16555) were purchased from Wako Pure Chemical Industries, Ltd. PdCl₂(dppf)·CH₂Cl₂ (Cat. No. B2064) was purchased from TOKYO CHEMICAL INDUSTRY CO., LTD.

Further, (Ph₃P)₄Pd (Cat. No. 216666) and (n-Bu₃Sn)₂ (Cat. No. 251127), Compound (13) (4-iodobenzyl alcohol) (Cat. No. 523496), 3-methylbenzyl bromide (Cat. No. B83509), and (diethylamino)sulfur trifluoride (Cat. No. 235253) were purchased from Sigma-Aldrich Japan. n-hexane solution of n-butyllithium (Cat. No. 04937-25) and potassium bromide (Cat. No. 32319-30), potassium acetate (32299-30), and triphenylphosphine (Cat. No. 40950-25) were purchased from KANTO CHEMICAL CO., INC.

All other chemical reagents used here were those of commercially available grades.

Further, Compound (3) (4-(4H-1,2,4-Triazol-4-ylamino)benzonitrile) was synthesized by reacting Compound (1) with Compound (2) in the presence of potassium tert-butoxide in DMSO according to the method described in Non-patent Literature 3 (Okada, M.; Yoden, T.; Kawaminami, E.; Shimada, Y.; Kudoh, M.; Isomura, Y.; Shikama, H.; Fujikura, T., Chem. Pharm. Bull. 1996, 44, 1871-1879). Compounds (9), (31), and (37) were synthesized according to the method described in Non-patent Literature 3.

### [Synthesis of compound]

### (I) Compound (4) (4-[4H-1,2,4-Triazol-4-yl){4-(tributylstannyl)benzyl}amino]benzonitrile)

### <Method A>

Compound (4) was synthesized according to the scheme below.

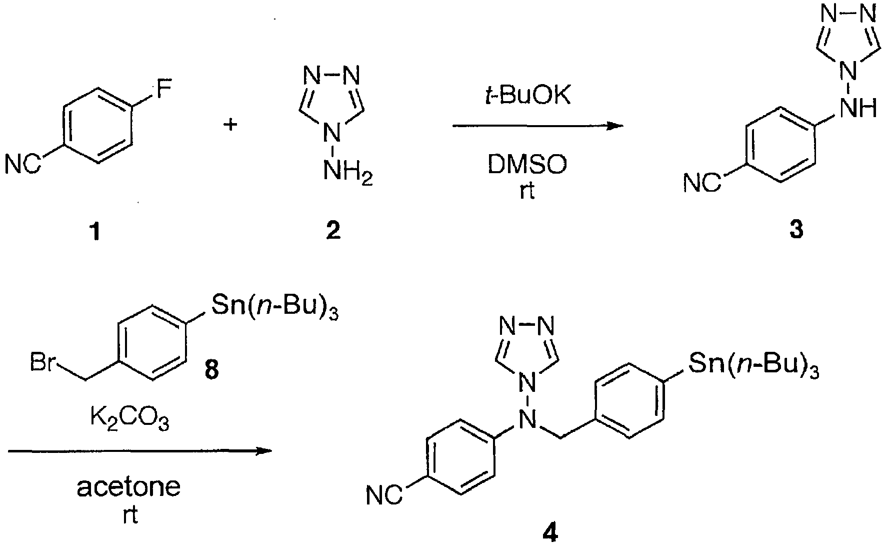

Here, Compound (8) was synthesized according to the scheme below.

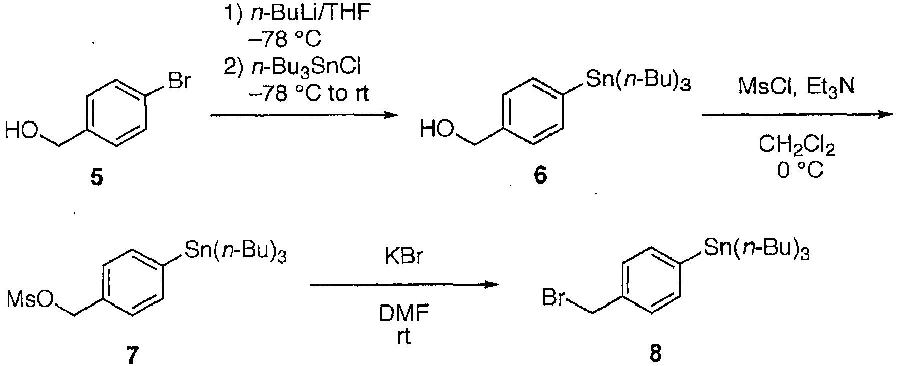

The following specifically explains how to synthesize individual compounds.

### (i) Synthesis of Compound (6) (4-(Tributylstannyl)benzyl alcohol) (see Non-patent Literature 11 (Huang, Y.; Hammond, P. S.; Wu, L.; Mach, R. H., J. Med. Chem. 2001, 44, 4404-4415), Non-patent Literature 12 (Kopka, K.; Faust, A.; Keul, P.; Wagner, S.; Breyholz, H.-J.; Holtke, C.; Schober, O.; Schafers, M.; Levkau, B..J. Med. Chem. 2006, 49, 6704-6715))

Under argon atmosphere, n-hexane solution of n-butyllithium (1.63 M, 30.0 mL, 48.9 mmol) was slowly added to anhydrous THF (150 mL) solution of Compound (5) (4.15 g, 22.2 mmol) at -78 °C. The mixture was stirred for 50 min at the same temperature, and then tributyltin(IV) chloride (13.3 mL, 48.9 mmol) was added to the mixture at -78 °C and the mixture was warmed up to room temperature. The reaction mixture was stirred for 1.5 hours and then concentrated under reduced pressure.

Water (200 mL) was added to the residue, and the mixture was extracted using CH₂Cl₂ (80 mL × 2). All the organic phases were mixed, sequentially rinsed with water (150 mL × 3) and a saline solution (150 mL × 1), dried (Na₂SO₄), filtered, and concentrated under reduced pressure.

The residue was purified by column chromatography (200 g of silica gel, n-hexane/EtOAc = 8/1) to obtain Compound (6) (colorless oily substance, 7.14 g, 81.0%).
TLC R_{f} = 0.44 (n-hexane/EtOAc = 4/1), R_{f} = 0.50 (n-hexane/CH₂Cl₂/EtOAc = 4/1/1)
¹H NMR (300 MHz, CDCl₃) δ 0.88 (t, 9H, J = 7.2 Hz, 3CH₃), 0.93-1.16 (m, 6H, 3CH₂), 1.26-1.39 (m, 6H, 3CH₂), 1.43-1.67 (m, 6H, 3CH₂), 4.68 (d, 2H, J = 5.8 Hz, benzylic CH₂), 7.30-7.37 (AA'BB', 2H, aromatic), 7.39-7.56 (AA'BB', 2H, ³J (^{119/117}Sn-¹H) = 37.4 Hz, aromatic)
¹³C NMR (75.5 MHz, CDCl₃) δ 9.3 (3C, ¹J (¹¹⁹Sn-¹³C) = 339.2 Hz, ¹J (¹¹⁷Sn-¹³C) = 324.5 Hz), 13.5 (3C), 27.2 (3C, ³J (^{119/117}Sn-¹³C) = 54.7 Hz), 28.9 (3C, ²J (^{119/117}Sn-¹³C) = 19.7 Hz), 64.2, 126.5 (2C, ³J (^{119/117}Sn-¹³C) = 41.3 Hz), 136.2 (2C, ²J (^{119/117}Sn-¹³C) = 31.4 Hz), 140.2, 140.5
IR (KBr, cm⁻¹) 514, 598, 621, 662, 689, 743, 791, 833, 864, 961, 1015, 1069, 1207, 1290, 1375, 1393, 1418, 1462, 2851, 2870, 2924, 2955, 3296
Anal. Calcd. for C₁₉H₃₄Sn: C, 57.46; H, 8.63. Found: C, 57.29; H, 8.88
HRMS (FAB⁺/NBA+NaI) m/z 421.1536 ([M+Na]⁺, C₁₉H₃₄O¹²⁰SnNa requires 421.1529).

### (ii) Synthesis of Compound (7) (4-(Tributylstannyl)benzyl methanesulfonate) (see Non-patent Literature 12)

Under argon atmosphere, triethylamine (3.63 mL, 26.0 mmol) and methanesulfonyl chloride (1.61 mL, 20.8 mmol) were sequentially added to CH₂Cl₂ (100 mL) solution of Compound (6) (6.90 g, 17.4 mmol) at 0 °C and the mixture was stirred for 35 min at the same temperature.

Water (200 mL) was added to the mixture, and the mixture was extracted using CH₂Cl₂ (80 mL × 3). All the organic phases were mixed, rinsed with a saline solution (150 mL × 3), dried (Na₂SO₄), filtered, and concentrated under reduced pressure. The residue was purified by column chromatography (200 g of silica gel, n-hexane/EtOAc = 4/1) to obtain Compound (7) (colorless oily substance, 7.17 g, 86.8%).
TLC R_{f} = 0.45 (n-hexane/EtOAc = 4/1), R_{f} = 0.57 (n-hexane/CH₂Cl₂/EtOAc = 4/1/1)
¹H NMR (300 MHz, CDCl₃) δ 0.88 (t, 9H, J = 7.2 Hz, 3CH₃), 0.94-1.19 (m, 6H, 3CH₂), 1.23-1.41 (m, 6H, 3CH₂), 1.42-1.67 (m, 6H, 3CH₂), 2.90 (s, 3H, CH₃), 5.23 (s, 2H, benzylic CH₂), 7.33-7.41 (AA'BB', 2H, aromatic), 7.43-7.60 (AA'BB', 2H, ³J(^{119/117}Sn-¹H) = 36.5 Hz, aromatic)
¹³C NMR (75.5 MHz, CDCl₃) δ 7.0 (3C, ¹J(¹¹⁹Sn-¹³C) = 340.7 Hz, ¹J(¹¹⁷Sn-¹³C) = 325.5 Hz), 13.3 (3C), 26.9 (3C, ³J(^{199/117}Sn-¹³C) = 55.4 Hz), 28.7 (3C, ²J(^{119/117}Sₙ-¹³C) = 20.0 Hz), 37.3, 71.4, 127.8 (2C, ³J^{(119/117}Sn-¹³C) = 40.3 Hz), 132.9, 136.4 (2C, ²J(^{119/117}Sn-¹³C) = 30.8 Hz), 143.1
IR (KBr, cm⁻¹) 513, 529, 664, 691, 816, 862, 932, 1069, 1175, 1356, 1396, 1416, 1464, 2851, 2870, 2924, 2955
Anal. Calcd. for C₂₀H₃₆O₃SSn: C, 50.54; H, 7.63. Found: C, 50.32; H, 7.80
HRMS (FAB⁺/NBA+NaI) m/z 499.1301 ([M+H]⁺, C₂₀H₃₆O₃S¹²⁰SnNa requires 499.1305).

### (iii) Synthesis of Compound (8) (4-(Tributylstannyl)benzyl bromide) (see Non-patent Literature 13 (Patel, H. K.; Kilburn, J. D.; Langley, G. J.; Edwards, P. D.; Mitchell, T.; Southgate, R., Tetrahedron Lett. 1994, 35, 481-484))

Under argon atmosphere, potassium bromide (3.46 g, 29.1 mmol) was added to a DMF (100 mL) solution of Compound (7) (6.91 g, 14.5 mmol) at room temperature, and the mixture was stirred at the same temperature for 12 hours.

Water (100 mL) was added to the mixture, and the mixture was extracted using Et₂O (80 mL × 3). All the organic phases were mixed, sequentially rinsed with water (150 mL × 3) and a saline solution (150 mL × 1), dried (Na₂SO₄), filtered, and concentrated under reduced pressure. The residue was purified by column chromatography (200 g of silica gel, n-hexane/Et₂O = 49/1) to obtain Compound (8) (colorless oily substance, 6.43 g, 96.1%).
TLC R_{f} = 0.39 (n-hexane)
¹H NMR (300 MHz, CDCl₃) δ 0.88 (t, 9H, J = 7.2 Hz, 3CH₃), 0.92-1.18 (m, 6H, 3CH₂), 1.25-1.40 (m, 6H, 3CH₂), 1.42-1.64 (m, 6H, 3CH₂), 4.49 (s, 2H, benzylic CH₂), 7.30-7.39 (AA'BB', 2H, aromatic), 7.39-7.54 (AA'BB', 2H, 3J(^{119/117}Sn-¹H) = 36.6 Hz, aromatic)
¹³C NMR (75.5 MHz, CDCl₃) δ 7.3 (3C, ¹J(¹¹⁹Sn-¹³C) = 340.0 Hz, ¹J(¹¹⁷Sn-¹³C) = 324.8 Hz), 13.6 (3C), 27.3 (3C, ³J(^{119/117}Sn-¹³C) = 55.4 Hz), 29.0 (3C,²J(^{119/117}Sn-¹³C) = 19.3 Hz), 33.4, 128.2 (2C,³J(^{119/117}Sn-¹³C) = 41.2 Hz), 136.6 (2C,²J(^{119/117}Sn-¹³C) = 30.5 Hz), 137.2, 142.2
IR (KBr, cm⁻¹) 627, 669, 777, 797, 839, 937, 1011, 1086, 1113, 1406, 1462, 1485, 2857, 2884, 2928, 2953
Anal. Calcd. for C₁₉H₃₃BrSn: C, 49.60; H, 7.23. Found: C, 49.44; H, 7.41.

### (iv) Synthesis of Compound (4) (4-[(4H-1,2,4-Triazol-4-yl){4-(tributylstannyl)benzyl}amino]benzonitrile)

Under argon atmosphere, an acetone (50 mL) solution of a mixture of Compound (3) (1.17 g, 6.33 mmol), Compound (8) (3.49 g, 7.59 mmol), and potassium carbonate (1.76 g, 12.7 mmol) was stirred at room temperature for 8 hours. Water (100 mL) was added to the mixture, and the mixture was extracted using CH₂Cl₂ (100 mL × 3). All the organic phases were mixed, sequentially rinsed with water (100 mL × 1) and a saline solution (100 mL × 1), dried (Na₂SO₄), filtered, and concentrated under reduced pressure.

The residue was purified by column chromatography (110 g of silica gel, CH₂Cl₂/CH₃OH = 20/1) to obtain Compound (4) (colorless solid, 2.62 g, 73.5%). Recrystallization of Compound (4) with n-hexane resulted in colorless plate crystal (2.26 g, 63.4%).
TLC R_{f} = 0.57 (CH₂Cl₂/CH₃OH = 9/1)
Mp 93-94 °C
¹H NMR (300 MHz, CDCl₃) δ 0.88 (t,9H,J = 7.2 Hz, 3CH₃), 0.93-1.18 (m, 6H, 3CH₂), 1.23-1.41 (m, 6H, 3CH₂), 1.44-1.58 (m, 6H, 3CH₂), 4.88 (s, 2H, benzylic CH₂), 6.62-6.70 (AA'BB', 2H,aromatic), 7.09-7.19 (AA'BB', 2H, aromatic), 7.36-7.53 (AA'BB', 2H, ³J(^{119/117}Sn-¹H) = 36.0 Hz, aromatic), 7.55-7.62 (AA'BB', 2H, aromatic), 8.12 (br s, 2H, triazole)
¹³C NMR (75.5 MHz, DMSO-d₆) δ 9.1 (3C, ¹J(¹¹⁹Sn-¹³C) = 338.8 Hz, ¹J(¹¹⁷Sn-¹³C) = 324.2 Hz), 13.6 (3C), 26.7 (3C, 3^{J}(^{119/117}Sn-¹³C) = 53.7 Hz), 28.6 (3C, ²J(^{119/117}Sn-¹³C) = 20.3 Hz), 57.3, 102.8, 113.6 (2C), 119.1, 127.9 (2C, ³J(^{119/117}Sn-¹³C) = 40.9 Hz), 133.9 (2C), 134.7, 136.4 (2C,²J(^{119/117}Sn-¹³C) = 31.9 Hz), 141.0, 143.3 (2C), 151.5
IR (KBr, cm⁻¹) 669, 826, 1065, 1179, 1292, 1335, 1395, 1462, 1508, 1605,2222,2851,2870,2924,2955
Anal. Calcd. for C₂₈H₃₉N₅Sn: C, 59.59; H, 6.97; N, 12.41. Found: C, 59.47; H, 6.65; N, 12.44
HRMS (FAB⁺/NBA) m/z 566.2318 ([M+H]⁺, C₂₈H₄₀N₅¹²⁰Sn requires 566.2306).

### <Method B>

Further, Compound (4) was also synthesized according to another method represented by the scheme below.

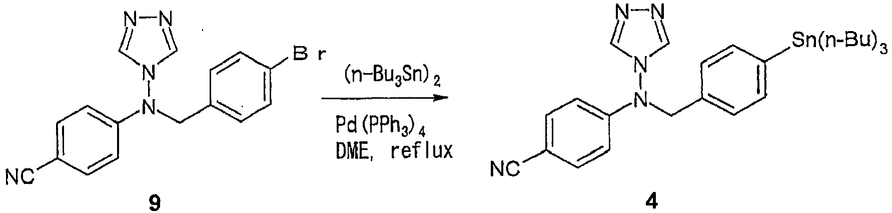

Specifically, Compound (4) was also synthesized as follows.

Compound (9) (4-{(4-Bromobenzyl)(4H-1,2,4-triazol-4-yl)amino}benzonitrile (YM511)) was synthesized by reacting Compound (3) with 4-bromobenzyl bromide in the presence of potassium carbonate in acetonitrile according to the method described in Patent Literature 1 (Okada, M.; Yoden, T.; Kawaminami, E.; Shimada, Y.; Kudoh, M.; Isomura, Y.; Shikama, H.; Fujikura, T., Chem. Pharm. Bull. 1996, 44, 1871-1879).

Initially, under argon atmosphere, (Ph₃P)₄Pd (66.9 mg, 57.9 µmol) and (n-Bu₃Sn)₂ (2.50 mL, 2.93 mmol) were sequentially added to a DME (20 mL) solution of Compound (9) (685 mg, 1.93 mmol) and the mixture was refluxed at 100 °C for 20 hours.

After cooling the mixture to room temperature, an aqueous solution of saturated potassium fluoride (50 mL) was added to the mixture, and the resulting precipitate was removed by filtration. The filtrate was extracted with ethyl acetate (100 mL × 2) and all the organic phases were mixed. The mixture was sequentially rinsed with a saturated potassium fluoride aqueous solution (100 mL × 1), water (100 mL × 1) and a saline solution (100 mL × 1), dried (Na₂SO₄), filtered, and concentrated under reduced pressure.

The residue was purified by column chromatography (50 g of silica gel, CH₂Cl₂/EtOAc = 9/1) to obtain Compound (4) (colorless solid, 551 mg, 50.5%).

### (II) Synthesis of Compound (10) (4-{(4-[¹¹C]Methylbenzyl)(4H-1,2,4-triazol-4-yl)amino}benzonitrile, [¹¹C]cetrozole)

Compound (10) was synthesized as follows.

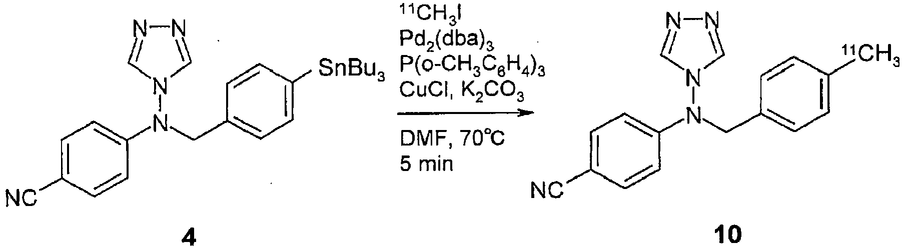

Initially, a DMF solution (0.3 mL) of trisdibenzylideneacetonedipalladium (1.8 mg, 1.6 µmol) and tri-o-tolylphosphine (2.0 mg, 6.3 µmol) was prepared in a reaction vessel (A) and left at room temperature. The solution in the reaction vessel (A) was prepared 10-20 min before injecting [¹¹C]methyl iodide into the reaction vessel (A).

On the other hand, the synthesized Compound (4) (4.0 mg, 13 µmol), CuCl (2.0 mg, 20 µmol), and K₂CO₃ (2.4 mg, 18 µmol) were prepared in a reaction vessel (B) and left at room temperature.

Subsequently, [¹¹C]methyl iodide was injected into the reaction vessel (A), which was then left for 1 min. The resulting solution was added to the reaction vessel (B).

The mixed solution in the reaction vessel (B) was heated at 70 °C for 5 min, and then subjected to separation and purification using HPLC (high-performance liquid chromatography) under conditions below.

Conditions for separation and purification: guard column Pd pack, AR-II 10 x 50 mm, preparative column Cholester Waters 10 x 250 mm, UV detection wavelength 254 nm, eluate CH₃CN:30 mM ammonium acetate = 40:60, flow rate 5.0 mL/min, retention time approximately 14 min, yield by HPLC of Compound (10) 91% or higher (calculated from area ratio of HPLC radioactive spectrum), total radioactivity of isolated (10) 2.3 GBq; Conditions for analysis (purification measurement, specific radioactivity measurement): AR-II 4.6 x 100 mm, UV detection wavelength 254 nm, eluate CH₃CN:30 mM ammonium acetate = 40:60, flow rate 1.0 mL/min, retention time approximately 4.5 min, radiochemical purity 99%, chemical purity 96-99%, specific radioactivity 72 GBq/µmol.
Diluting solution: 5.0 mL of physiological saline, 0.5 mL of propylene glycol, and 0.08 mL of Tween 80 surfactant.

Fig. 1 shows the result of HPLC preparation of ¹¹C-labeled compound (10). In Fig. 1, the peak whose base line is positioned at the lower side was detected by RI detection, and the peak whose base line is positioned at the upper side was detected by UV detection.

After separation by HPLC, the fraction containing the ¹¹C-labeled Compound (10) was concentrated by an evaporator under reduced pressure, and a diluting solution was added to the concentrated fraction to obtain an administration solution for clinical research on monkeys.

Further, Compound (10) was synthesized also as follows.

Using a cyclotron (product name; Sumitomo CYPRIS HM-12S cyclotron, manufactured by Sumitomo Heavy Industries, Ltd.), [¹¹C]carbon dioxide was synthesized by ¹⁴N(p,α)¹¹C reaction, and then lithium aluminum hydride and hydriodic acid were added in this order by a labeling synthesis device (RIKEN original automated radiolabeling system) so that the resultant was converted into [¹¹C]methyl iodide. The resulting [¹¹C]methyl iodide was used in a high-speed C-[¹¹C]methylation reaction using palladium(0) catalyst shown below.

[¹¹C]Methyl iodide was transferred by He gas flow (30 mL/min) to a DMF (0.3 mL) solution of Pd₂(dba)₃ (2.0 mg, 1.8 µmol) and P(o-tolyl)₃ (3.2 mg, 10.1 µmol) in the reaction vessel (A) at room temperature. The resulting mixture was transferred to the reaction vessel (B) containing the Compound (4) (2.5 mg, 4.4 µmol), CuCl (2.0 mg, 20 µmol), and K₂CO₃ (2.5 mg, 19 µmol).

The inside of the reaction vessel (A) was rinsed with DMF (0.5 mL) and the resulting solution was transferred to the reaction vessel (B).

The resulting mixture was heated at 70 °C for 5 min. The salts and palladium residues in the reaction mixture were removed by solid-phase extraction and rinsing with 1 mL of CH₃CN:30 mM CH₃COONH₄ (35:65) solution.

The combined eluate was injected into a preparative HPLC equipped with a γ detector (mobile phase, CH₃CN:30 mM CH₃COONH₄ (35:65); column, Nacalai COSMOSIL AR-II C18, 10 mm × 250 mm, 5 mm; guard column, Sumika SUMIPAK Filter PG-ODS; flow rate, 6 mL/min; UV detection wavelength, 254 nm; retention time of Compound (10), 13.5 min).

The fraction of interest was put in a flask and the organic solvent was removed under reduced pressure. The ¹¹C-labeled compound of interest was dissolved in a mixture of polysorbate 80 (0.05 mL), propylene glycol (0.3 mL) and physiological saline (4 mL).

The total time for all synthetic processes including HPLC purification and preparation of radioactive drug for intravenous administration was 38 min.

The Compound (10) prepared for administration exhibited radioactivity of 1.5-2.3 GBq and specific radioactivity of 50-120 GBq/µmol.

The Compound (10) was identified by injecting, together with Compound (11) which is a non-radiolabeled authentic sample, into an analytical HPLC equipped with a γ detector (mobile phase, CH₃CN:30 mM CH₃COONH₄ (40:60); column, Nacalai COSMOSIL AR-II C18, 4.6 mm × 100 mm; flow rate, 1 mL/min; UV detection wavelength, 254 nm; retention time of Compound (10), 4.5 min). The chemical purity and the radiochemical purity were 95% or higher.

### (III) Synthesis of Compound (11) (4-{(4-Methylbenzyl)(4H-1,2,4-triazol-4-yl)amino)benzonitrile (cetrozole))

As a control for the Compound (10), Compound (11) which is not radiolabeled was synthesized according to the scheme below.

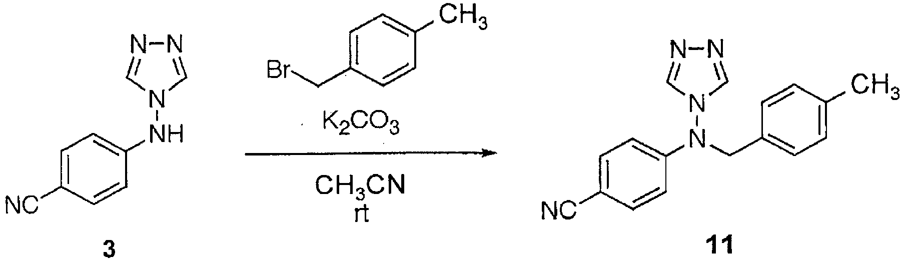

Under argon atmosphere, an acetonitrile (80 mL) solution of a mixture of Compound (3) (2.00 g, 10.8 mmol), 4-methylbenzyl bromide (Cat. No. 021-03131, purchased from Wako Pure Chemical Industries, Ltd) (2.00 g, 10.8 mmol), and potassium carbonate (2.99 g, 21.6 mmol) was stirred at room temperature for 2 hours. Water (200 mL) was added to the solution, and the mixture was extracted with CH₂Cl₂ (100 mL × 3). All the organic phases were mixed, rinsed with water (30 mL × 2), dried (Na₂SO₄), filtered, and concentrated under reduced pressure.

The residue was purified by column chromatography (200 g of silica gel, CH₂Cl₂/EtOAc = 1/1-1/4) to obtain Compound (11) (colorless solid, 1.60 g, 51.2%). Recrystallization of the Compound (11) with ethyl acetate (64 mL) resulted in colorless plate crystal (1.27 g, 40.6%).
TLC R_{f}= 0.30 (CH₂Cl₂/EtOAc = 1/1)
Mp 201-202 °C
¹H NMR (300 MHz, DMSO-d₆) δ 2.25 (s,3H,CH₃), 4.99 (s, 2H, benzylic CH₂), 6.70-6.78 (AA'BB', 2H, aromatic), 7.06-7.14 (AA'BB', 2H, aromatic), 7.14-7.22 (AA'BB', 2H,aromatic), 7.70-7.79 (AA'BB', 2H, aromatic), 8.76 (br s, 2H, triazole)
¹³C NMR (75.5 MHz, DMSO-d₆) δ 20.7, 56.9, 102.8, 113.7 (2C), 119.1, 128.5 (2C), 129.3 (2C), 131.2, 133.9 (2C), 137.4, 143.4 (2C), 151.6;
IR (KBr, cm⁻¹) 548, 606, 667, 741, 814, 833, 858, 870, 1069, 1180, 1207, 1234, 1265, 1287, 1302, 1389, 1456, 1501, 1512, 1605, 2220, 2340, 2359, 3121
Anal. Calcd. for C₁₇H₁₅N₅: C, 70.57; H, 5.23; N, 24.21. Found: C, 70.41; H, 5.10; N, 24.48
HRMS (FAB⁺/NBA+NaI) m/z 312.1220 ([M+Na]⁺, C₁₇H₁₅N₅Na requires 312.1225).

### (IV) Synthesis of Compound (12) (4-[{4-(4,4,5,5-Tetramethyl-1,3,2-dioxaborolan-2-yl)benzyl}(4H-1,2,4-triazol-4-yl)amino]benzonitrile)

### <Method A>

Compound (12) was synthesized according to the scheme below.

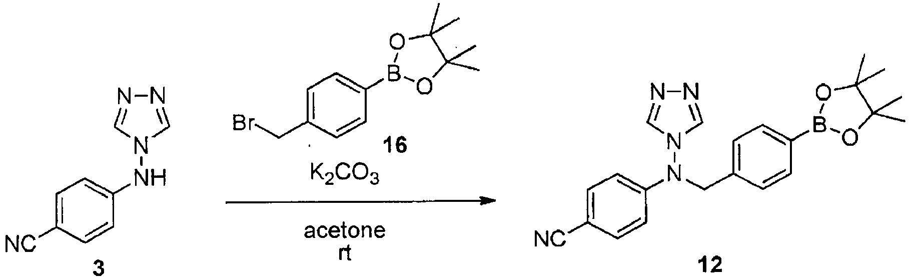

Here, Compound (16) was synthesized according to the scheme below.

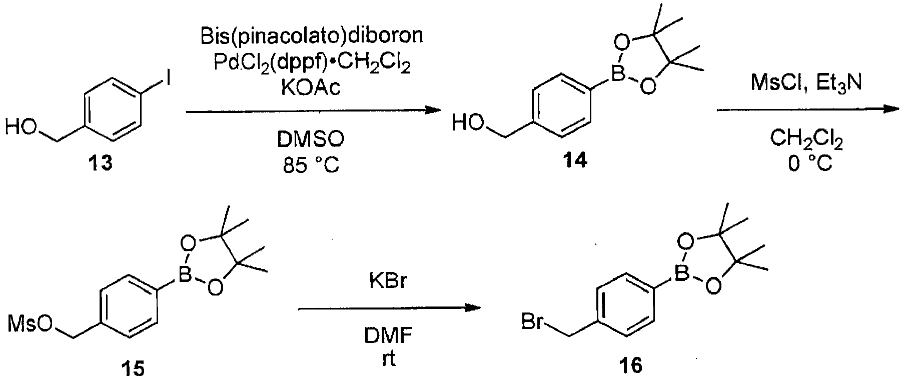

### (i) Synthesis of Compound (14) (4-(4,4,5,5-Tetramethyl-1,3,2-dioxaborolan-2-yl)benzyl alcohol) (see Non-patent Literature 14 (Filippis, A.; Morin, C.; Thimon, C., Synth. Commun. 2002, 32, 2669-2676))

Under argon atmosphere, PdCl₂(dppf)·CH₂Cl₂ (245 mg, 300 µmol), potassium acetate (2.94 g, 30.0 mmol) and bis(pinacolato)diboron (2.79 g, 11.0 mmol) were sequentially added to a DMSO (30 mL) solution of Compound (13) (2.34 g, 10.0 mmol) at room temperature, and the mixture was stirred at 85 °C for 18 hours.

The mixture was cooled to room temperature, and then water (250 mL) was added to the mixture and the mixture was extracted with ethyl acetate (100 mL × 3).

All the organic phases were mixed, sequentially rinsed with water (100 mL × 3) and a saline solution (100 mL × 1), dried (Na₂SO₄), filtered, and concentrated under reduced pressure.

The residue was purified by flash column chromatography (60 g of silica gel, n-hexane/EtOAc = 5/1) to obtain Compound (14) as a colorless solid.
TLC R_{f} = 0.41 (n-hexane/EtOAc = 2/1)
¹H NMR (300 MHz, CDCl₃) δ 1.35 (s, 12H, 4CH₃), 4.72 (s, 2H, benzylic CH₂), 7.34-7.41 (AA'BB', 2H, aromatic), 7.78-7.84 (AA'BB', 2H, aromatic).

### (ii) Synthesis of Compound (15) (4-(4,4,5,5-Tetramethyl-1,3,2-dioxaborolan-2-yl)benzyl methanesulfonate) (see Non-patent Literature 14)

Under argon atmosphere, triethylamine (3.15 mL, 22.6 mmol) and methanesulfonyl chloride (1.40 mL, 18.1 mmol) were sequentially added to a CH₂Cl₂ (60 mL) solution of Compound (14) (3.48 g, 14.9 mmol), and the mixture was stirred at 0 °C for 2 hours. Water (150 mL) was added to the mixture, and the mixture was extracted with CH₂Cl₂ (100 mL × 3). All the organic phases were mixed, sequentially rinsed with water (70 mL × 3) and a saline solution (70 mL × 3), dried (Na₂SO₄), filtered, and concentrated under reduced pressure.

The residue was purified by flash column chromatography (110 g of silica gel, n-hexane/EtOAc = 3/1) to obtain Compound (15) as a colorless solid. The Compound (15) was not purified furthermore and used in the next step. TLC 0.41 (n-hexane/EtOAc = 2/1).

### (iii) Synthesis of Compound (16) (4-(4,4,5,5-Tetramethyl-1,3,2-dioxaborolan-2-yl)benzyl bromide) (see Non-patent Literature 14)

Under argon atmosphere, potassium bromide (2.03 g, 17.1 mmol) was added to a DMF (25 mL) solution of Compound (15) (3.55 g, 11.4 mmol) at room temperature, and the mixture was stirred at room temperature for 19 hours. Water (150 mL) was added to the mixture, and the mixture was extracted with ethyl acetate (100 mL × 3). All the organic phases were mixed, rinsed with water (100 mL × 3), dried (Na₂SO₄), filtered, and concentrated under reduced pressure.

The residue was purified by flash column chromatography (100 g of silica gel, n-hexane/EtOAc = 9/1) to obtain Compound (16) as a colorless solid.
TLC R_{f} = 0.73 (n-hexane/EtOAc = 3/1)
¹H NMR (300 MHz, CDCl₃) δ 1.34 (s, 12H, 4CH₃), 4.49 (s, 2H, benzylic CH₂), 7.36-7.43 (AA'BB', 2H, aromatic), 7.75-7.83 (AA'BB', 2H, aromatic).

### (iv) Synthesis of Compound (12)

Under argon atmosphere, an acetone (50 mL) solution of a mixture of Compound (3) (1.36 g, 7.33 mmol), Compound (16) (2.61 g, 8.79 mmol) and potassium carbonate (2.03 g, 14.7 mmol) was stirred at room temperature for 5 hours. Water (150 mL) was added to the mixture, and the mixture was extracted with ethyl acetate (100 mL × 3). All the organic phases were mixed, sequentially rinsed with water (50 mL × 1) and a saline solution (50 mL × 1), dried (Na₂SO₄), filtered, and concentrated under reduced pressure.

The residue was purified by flash column chromatography (110 g of silica gel, CH₂Cl₂/CH₃OH = 40/1) to obtain Compound (12) (colorless solid, 2.02 g, 68.5%).
TLC R_{f} = 0.57 (CH₂Cl₂/CH₃OH = 9/1)
Mp 193-194 °C
¹H NMR (300 MHz, CDCl₃) δ 1.34 (s, 12H, 4CH₃), 4.91 (s, 2H, benzylic CH₂), 6.63-6.70 (AA'BB', 2H,aromatic), 7.18-7.25 (AA'BB', 2H, aromatic), 7.55-7.63 (AA'BB', 2H, aromatic), 7.75-7.83 (AA'BB', 2H, aromatic), 8.11 (s, 2H, triazole)
¹³C NMR (67.8 MHz, CDCl₃) δ 24.7 (4C), 58.2, 83.9 (2C), 104.8, 113.3 (2C), 118.5, 127.2 (2C), 133.9 (2C), 135.5 (2C), 136.3, 142.5 (2C), 150.4 (the carbon adjacent to boron was not observed)
IR (KBr, cm⁻¹) 546, 656, 671, 733, 826, 858, 912, 962, 1020, 1065, 1088, 1142, 1179, 1213, 1271, 1325, 1360, 1398, 1508, 1603, 2222, 2978, 3115, 3401
HRMS (ESI⁺) m/z 424.1912 ([M+Na]⁺, C₂₂H₂₄BN₅NaO₂⁺ requires 424.1915).

### <Method B>

Further, the Compound (12) was also synthesized according to another method represented by the scheme below.

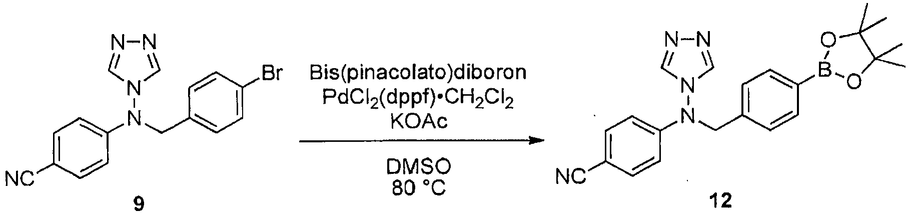

Specifically, the Compound (12) was synthesized as follows.

Under argon atmosphere, PdCl₂(dppf)·CH₂Cl₂ (22.5 mg, 27.5 µmol), potassium acetate (270 mg, 2.75 mmol) and bis(pinacolato)diboron (256 mg, 1.01 mmol) were sequentially added to a DMSO (5.5 mL) solution of Compound (9) (324 mg, 915 µmol) at room temperature, and the mixture was stirred at 80 °C for 2 hours. The mixture was cooled to room temperature, and then water (80 mL) was added to the mixture, and the mixture was extracted with ethyl acetate (50 mL × 3). All the organic phases were mixed, sequentially rinsed with water (50 mL × 3) and a saline solution (50 mL × 3), dried (Na₂SO₄), filtered, and concentrated under reduced pressure.

The residue was purified by flash column chromatography (30 g of silica gel, CH₂Cl₂/CH₃OH = 20/1) to obtain Compound (12) (pale gray solid, 330 mg, 89.9%).

### (V) Synthesis of Compound (10) from Compound (12)

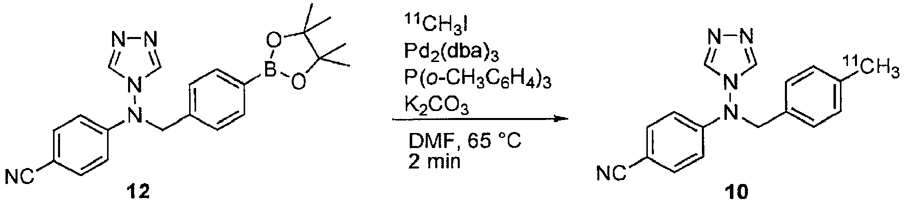

Using a cyclotron (product name; Sumitomo CYPRIS HM-12S cyclotron, manufactured by Sumitomo Heavy Industries, Ltd.), [¹¹C]carbon dioxide was synthesized by ¹⁴N(p,α)¹¹C reaction, and then lithium aluminum hydride and hydriodic acid were added in this order by a labeling synthesis device (RIKEN original automated radiolabeling system) so that the resultant was converted into [¹¹C]methyl iodide. The resulting [¹¹C]methyl iodide was used in a high-speed C-[¹¹C]methylation reaction using palladium(0) catalyst shown below.

[¹¹C]Methyl iodide was transferred by He gas flow (30 mL/min) to a DMF (0.5 mL) solution of Pd₂(dba)₃ (2.9 mg, 2.5 µmol), P(o-tolyl)₃ (4.0 mg, 12.6 µmol) and K₂CO₃ (4.0 mg, 30 µmol) at room temperature.

The resulting mixture was heated at 65 °C for 2 min. The salt and palladium residues in the reaction mixture were removed by solid-phase extraction and rinsing with 1 mL of CH₃CN:30 mM CH₃COONH₄ (35:65) solution.

The combined eluate was poured into a preparative HPLC equipped with a □ detector (mobile phase, CH₃CN:30 mM CH₃COONH₄ (35:65); column, Nacalai COSMOSIL AR-II C18, 10 mm × 250 mm; guard column, Sumika SUMIPAK Filter PG-ODS; flow rate, 6 mL/min; UV detection wavelength, 254 nm; retention time of Compound (10), 13.5 min).

The fraction of interest was put in a flask and the organic solvent was removed under reduced pressure. The ¹¹C-labeled compound of interest was dissolved in a mixture of polysorbate 80 (0.05 mL), propylene glycol (0.3 mL) and physiological saline (4 mL).

The total time for all synthetic processes including HPLC purification and preparation of radioactive drug for intravenous administration was 30 min.

The Compound (10) prepared for administration exhibited radioactivity of 2.6-6.2 GBq and specific radioactivity of 90-110 GBq/µmol.

The Compound (10) was identified by injecting, together with Compound (11) which is a non-radiolabeled authentic sample, into an analytical HPLC equipped with a γ detector (mobile phase, CH₃CN:30 mM CH₃COONH₄ (40:60); column, Nacalai COSMOSIL AR-II C18, 4.6 mm × 100 mm; flow rate, 1 mL/min; UV detection wavelength, 254 nm; retention time of Compound (10), 4.5 min). The chemical purity and the radiochemical purity were 95% or higher.

### (VI) Synthesis of Compound (22) (4-{(3-[¹¹C]Methylbenzyl)(4H-1,2,4-triazol-4-yl)amino}benzonitrile)

Compound (22) was synthesized according to the scheme below.

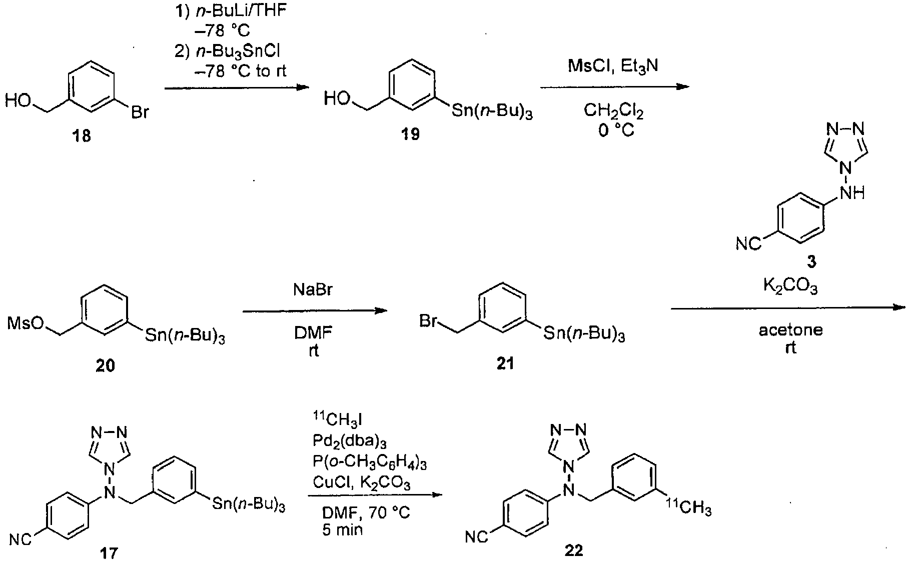

### (i) Synthesis of Compound (19) (3-(Tributylstannyl)benzyl alcohol) (see Non-patent Literature 15 (Efange, S. M. N.; Michelson, R. H.; Khare, A. B.; Thomas, J. R., J. Med. Chem. 1993, 36, 1754-1760))

Under argon atmosphere, n-hexane solution of n-butyllithium (1.63 M, 30.0 mL, 48.9 mmol) was slowly added to anhydrous THF (150 mL) solution of Compound (18) (2.66 mL, 22.2 mmol) at -78 °C. The reaction mixture was stirred at -78 °C for 30 min, and then tributyltin(IV) chloride (13.5 mL, 49.8 mmol) was added to the reaction mixture at -78 °C, which was warmed up to room temperature. The reaction mixture was stirred for 1 hour and then concentrated under reduced pressure. Water (200 mL) was added to the residue, which was extracted with CH₂Cl₂ (80 mL × 3).

All the organic phases were mixed, sequentially rinsed with water (100 mL × 3) and a saline solution (100 mL × 3), dried (Na₂SO₄), filtered, and concentrated under reduced pressure. The residue was purified by flash column chromatography (220 g of silica gel, n-hexane/EtOAc = 10/1) to obtain Compound (19) (colorless oily substance, 7.47 g, 84.8%).
TLC R_{f} = 0.52 (n-hexane/EtOAc = 4/1), R_{f} = 0.52 (n-hexane/CH₂Cl₂/EtOAc = 5/2/1)
¹H NMR (300 MHz, CDCl₃) δ 0.86 (t, 9H, J = 7.4 Hz, 3CH₃), 0.93-1.18 (m, 6H, 3CH₂), 1.22-1.44 (m, 6H, 3CH₂), 1.45-1.63 (m, 6H, 3CH₂), 4.67 (s, 2H, benzylic CH₂), 7.26-7.54 (m, 4H, aromatic).

### (ii) Synthesis of Compound (20) (3-(Tributylstannyl)benzyl methanesulfonate)

Under argon atmosphere, triethylamine (3.80 mL, 27.3 mmol) and methanesulfonyl chloride (1.66 mL, 21.5 mmol) were sequentially added to a CH₂Cl₂ (35 mL) solution of Compound (19) (7.12 g, 17.9 mmol), and the mixture was stirred at 0 °C for 3 hours. Water (100 mL) was added to the mixture, and the mixture was extracted with CH₂Cl₂ (80 mL × 3). All the organic phases were mixed, sequentially rinsed with water (50 mL × 1) and a saline solution (50 mL × 1), dried (Na₂SO₄), filtered, and concentrated under reduced pressure.

The residue was purified by flash column chromatography (220 g of silica gel, n-hexane/EtOAc = 9/1) to obtain Compound (20) (colorless oily substance, 4.85 g, 56.9%).
TLC 0.48 (n-hexane/EtOAc = 4/1), R_{f} = 0.65 (n-hexane/CH₂Cl₂/EtOAc = 5/2/1)
¹H NMR (300 MHz, CDCl₃) δ 0.89 (t, 9H, J = 7.3 Hz, 3CH₃), 0.94-1.20 (m, 6H, 3CH₂), 1.26-1.40 (m, 6H, 3CH₂), 1.46-1.59 (m, 6H, 3CH₂), 2.88 (s, 3H, CH₃), 5.24 (s, 2H, benzylic CH₂), 7.29-7.58 (m, 4H, aromatic)
¹³C NMR (75.5 MHz, CDCl₃) δ 9.5 (3C, ¹J(¹¹⁹Sn-¹³C) = 366.8 Hz, ¹J(¹¹⁷Sn-¹³C) = 341.7 Hz), 13.6 (3C), 27.2 (3C, ³J(^{119/117}Sn-¹³C) = 56.0 Hz), 28.9 (3C, ²J(^{119/117}Sn-¹³C) = 20.2 Hz), 38.2, 72.0, 128.2 (³J(^{119/117}Sn-¹³C) = 39.5 Hz), 128.6, 132.5 (³J(^{119/117}Sn-¹³C) = 38.9 Hz), 136.8 (²J(^{119/117}Sn-¹³C) = 30.8 Hz), 137.4 (²J(^{119/117}Sn-¹³C) = 28.7 Hz), 143.1
IR (KBr, cm⁻¹) 507, 529, 700, 783, 829, 918, 935, 1175, 1356, 1464, 2851,2870,2926,2957
Anal. Calcd. for C₂₀H₃₆O₃SSn: C, 50.54; H, 7.63. Found: C, 50.32; H, 7.28
HRMS (FAB⁺/NBA+NaI) m/z 499.1322 (M+H, C₂₀H₃₆O₃S¹²⁰SnNa requires 499.1305).

### (iii) Synthesis of Compound (21) (3-(Tributylstannyl)benzyl bromide)

Under argon atmosphere, sodium bromide (1.93 g, 18.8 mmol) was added to a DMF (30 mL) solution of Compound (20) (2.66 mL, 22.2 mmol) at room temperature, and the mixture was stirred at room temperature for 16 hours. Water (200 mL) was added to the mixture, and the mixture was extracted with diethyl ether (80 mL × 3). All the organic phases were mixed, sequentially rinsed with water (50 mL × 3) and a saline solution (50 mL × 1), dried (Na₂SO₄), filtered, and concentrated under reduced pressure.

The residue was purified by flash column chromatography (100 g of silica gel, n-hexane/Et₂O = 20/1) to obtain Compound (21) (colorless oily substance, 4.03 g, 93.3%).
TLC R_{f} = 0.68 (n-hexane)
¹H NMR (300 MHz, CDCl₃) δ 0.89 (t, 9H, J = 7.4 Hz, 3CH₃), 0.93-1.19 (m, 6H, 3CH₂), 1.25-1.41 (m, 6H, 3CH₂), 1.43-1.67 (m, 6H, 3CH₂), 4.50 (s, 2H, benzylic CH₂), 7.26-7.53 (m, 4H, aromatic)
¹³C NMR (75.5 MHz, CDCl₃) δ 9.5 (3C, ¹J(¹¹⁹Sn-¹³C) = 340.8 Hz, ¹J(¹¹⁷Sn-¹³C) = 325.7 Hz), 13.6 (3C), 27.3 (3C, ³J(^{119/117}Sn-¹³C) = 56.3 Hz), 29.0 (3C, ²J(^{119/117}Sn-¹³C) = 20.1 Hz), 33.9, 128.1 (³J(^{119/117}Sn-¹³C) = 40.3 Hz), 128.6, 136.4 (2J(^{119/117}Sn-¹³C) = 29.6 Hz), 136.8, 137.1 (³J(^{119/117}Sn-¹³C) = 38.8 Hz), 142.8
IR (KBr, cm⁻¹) 664, 700, 1125, 1462, 2851, 2870, 2924, 2955
Anal. Calcd. for C₁₉H₃₃BrSn: C, 49.60; H, 7.23. Found: C, 49.66; H,6.84.

### (iv) Synthesis of Compound (17) (4-[(4H-1,2,4-Triazol-4-yl){3-(tributylstannyl)benzyl}amino]benzonitrile)

Under argon atmosphere, an acetone (30 mL) solution of a mixture of Compound (3) (500 mg, 2.70 mmol), Compound (21) (1.49 g, 3.24 mmol), and potassium carbonate (746 mg, 5.40 mmol) was stirred at room temperature for 21 hours. The mixture was concentrated under reduced pressure. Water (100 mL) was added to the residue, and the residue was extracted using CH₂Cl₂ (70 mL × 3). All the organic phases were mixed, rinsed with water (50 mL × 1), dried (Na₂SO₄), filtered, and concentrated under reduced pressure.

The residue was purified by flash column chromatography (60 g of silica gel, CH₂Cl₂ to CH₂Cl₂/CH₃OH - 40/1) to obtain Compound (17) (colorless solid, 1.46 g, 95.8%). Recrystallization of the Compound (17) with n-hexane resulted in colorless plate crystal (1.13 g, 74.2%).
TLC R_{f} = 0.63 (CH₂Cl₂/EtOAc = 9/1)
Mp 116-117 °C
¹H NMR (300 MHz, CDCl₃) δ 0.87 (t, 9H, J = 7.3 Hz, 3CH₃), 0.92-1.16 (m, 6H, 3CH₂), 1.23-1.37 (m, 6H, 3CH₂), 1.42-1.56 (m, 6H, 3CH₂), 4.89 (s, 2H, benzylic CH₂), 6.63-6.71 (AA'BB', 2H, aromatic), 7.09 (d, 1H, J = 7.1 Hz, aromatic), 7.19-7.35 (m, 2H, aromatic), 7.36-7.53 (d, 1H, J = 7.1 Hz, ³J(^{119/117}Sn-¹H) = 36.1 Hz, aromatic), 7.55-7.63 (AA'BB', 2H, aromatic), 8.08 (s, 2H, triazole)
¹³C NMR (67.8 MHz, CDCl₃) δ 9.5 (3C, ¹J(¹¹⁹Sn-¹³C) = 342.1 Hz, ¹J(¹¹⁷Sn-¹³C) = 326.8 Hz), 13.6 (3C), 27.2 (3C, ³J(^{119/117}Sn-¹³C) = 56.0 Hz), 28.9 (3C, ²J(^{119/117}Sn-¹³C) = 20.2 Hz), 58.2, 104.7, 113.2 (2C), 118.5, 127.5, 128.5 (³J(^{119/117}Sn-¹³C) = 38.7 Hz), 132.8 (³J(^{119/117}Sn-¹³C) = 38.0 Hz), 133.9 (2C), 135.6 (²J (^{119/117}Sn-¹³C) = 30.0 Hz), 137.0 (²J(^{119/117}Sn-¹³C) = 28.5 Hz), 142.5 (2C), 143.9, 150.5
IR (KBr, cm⁻¹) 704, 837, 874, 1069, 1179, 1269, 1379, 1462, 1508, 1605,2224,2851,2870,2924,2957

Anal. Calcd. for C₂₈H₃₉N₅Sn: C, 59.59; H, 6.97; N, 12.41. Found: C, 59.55; H,6.81; N, 12.38.

### (v) Synthesis of Compound (22) (4-{(3-[¹¹C]Methylbenzyl)(4H-1,2,4-triazol-4-yl)amino}benzonitrile)

Using a cyclotron (product name; Sumitomo CYPRIS HM-12S cyclotron, manufactured by Sumitomo Heavy Industries, Ltd.), [¹¹C]carbon dioxide was synthesized by ¹⁴N(p,α)¹¹C reaction, and then lithium aluminum hydride and hydriodic acid were added in this order by a labeling synthesis device (RIKEN original automated radiolabeling system) so that the resultant was converted into [¹¹C]methyl iodide. The resulting [¹¹C]methyl iodide was used in a high-speed C-[¹¹C]methylation reaction using palladium(0) catalyst shown below.
[¹¹C]Methyl iodide was transferred by He gas flow (30 mL/min) to a DMF (0.3 mL) solution of Pd₂(dba)₃ (2.7 mg, 2.4 µmol) and P(o-tolyl)₃ (3.0 mg, 9.5 µmol) in the reaction vessel (A) at room temperature. The resulting mixture was transferred to the reaction vessel (B) containing the Compound (17) (3.0 mg, 5.3 mmol), CuCl (2.0 mg, 20 µmol), and K₂CO₃ (2.4 mg, 18 µmol). The inside of the reaction vessel (A) was rinsed with DMF (0.5 mL) and the resulting solution was transferred to the reaction vessel (B).

The resulting mixture was heated at 70 °C for 5 min. The salt and palladium residues in the reaction mixture were removed by solid-phase extraction and rinsing with 1 mL of CH₃CN:30 mM CH₃COONH₄ (40:60) solution.

The combined eluate was injected into a preparative HPLC equipped with a γ detector (mobile phase, CH₃CN:30 mM CH₃COONH₄ (40:60); column, Nacalai COSMOSIL AR-II C18, 10 mm × 250 mm; guard column, Nacalai COSMOSIL Cholester or Sumika SUMIPAK Filter PG-ODS; flow rate, 5 mL/min; UV detection wavelength, 254 nm; retention time of Compound (22), 12 min). The fraction of interest was put in a flask and the organic solvent was removed under reduced pressure.

The ¹¹C-labeled compound of interest was dissolved in a mixture of polysorbate 80 (0.05 mL), propylene glycol (0.3 mL) and physiological saline (4 mL).

The total time for all synthetic processes including HPLC purification and preparation of radioactive drug for intravenous administration was 38 min.

The Compound (22) prepared for administration exhibited radioactivity of 0.4-4.5 GBq and specific radioactivity of 30-60 GBq/µmol.

The Compound (22) was identified by injecting, together with Compound (23) which is a non-radiolabeled authentic sample, into an analytical HPLC equipped with a γ detector (mobile phase, CH₃CN:30 mM CH₃COONH₄ (40:60); column, Nacalai COSMOSIL AR-II C18, 4.6 mm × 100 mm; flow rate, 1 mL/min; UV detection wavelength, 254 nm; retention time of Compound (22), 4.6 min). The chemical purity and the radiochemical purity were 95% or higher.

### (VII) Synthesis of Compound (23) (4- {(3-Methylbenzyl)(4H-1,2,4-triazol-4-yl)amino}benzonitrile)

As a control for the Compound (22), Compound (23) which is not radiolabeled was synthesized according to the scheme below.

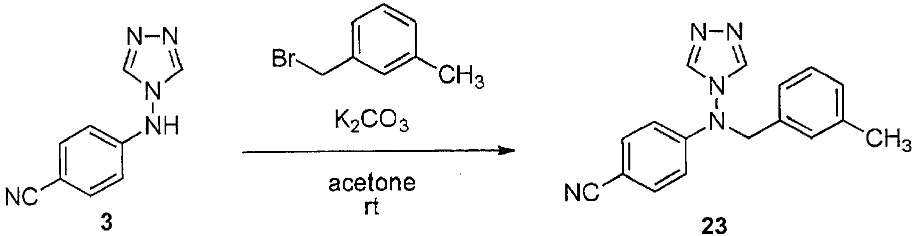

Under argon atmosphere, an acetone (15 mL) solution of a mixture of Compound (3) (300 mg, 1.62 mmol), 3-methylbenzyl bromide (265 µL, 1.96 mmol), and potassium carbonate (448 mg, 3.24 mmol) was stirred at room temperature for 5 hours. Water (50 mL) was added to the mixture, and the mixture was extracted using CH₂Cl₂ (50 mL × 3). All the organic phases were mixed, sequentially rinsed with water (50 mL × 1) and a saline solution (50 mL × 1), dried (Na₂SO₄), filtered, and concentrated under reduced pressure.

The residue was purified by flash column chromatography (30 g of silica gel, CH₂Cl₂/CH₃OH = 9/1) to obtain Compound (23) (colorless solid, 373 mg, 79.6%). Recrystallization of the Compound (23) with ethyl acetate resulted in colorless plate crystal (293 mg, 62.5%).
TLC R_{f} = 0.41 (CH₂Cl₂/CH₃OH = 9/1)
Mp 185-186 °C
¹H NMR (300 MHz, CDCl₃) δ 2.32 (s, 3H, CH₃), 4.86 (s, 2H, benzylic CH₂), 6.63-6.72 (AA'BB', 2H, aromatic), 6.95-7.15 (m, 2H, aromatic), 7.17 (d, 1H, J = 7.5 Hz, aromatic), 7.23 (d, 1H, J = 7.5 Hz, aromatic), 7.55-7.64 (AA'BB', 2H,aromatic), 8.10 (s, 2H, triazole)
¹³C NMR (67.8 MHz, DMSO-d₆) δ 20.9, 57.2, 102.7, 113.6 (2C), 119.1, 125.4, 128.5, 128.7, 128.8, 133.9 (2C), 134.7, 137.9, 143.4 (2C), 151.6
IR (KBr, cm⁻¹) 513, 546, 615, 648, 669, 704, 737, 766, 791, 827, 860, 949, 1007, 1040, 1069, 1140, 1182, 1213, 1300, 1321, 1391, 1425, 1460, 1510, 1605, 1692, 2218, 2868, 2918, 3011, 3051, 3100, 3121
Anal. Calcd. for C₁₇H₁₅N₅: C, 70.57; H, 5.23; N, 24.21. Found: C, 70.33; H, 5.30; N, 23.94.

### (VIII) Synthesis of Compound (24) (4-{(3-Bromobenzyl)(4H-1,2,4-triazol-4-yl)amino}benzonitrile) (see Non-patent Literature 3)

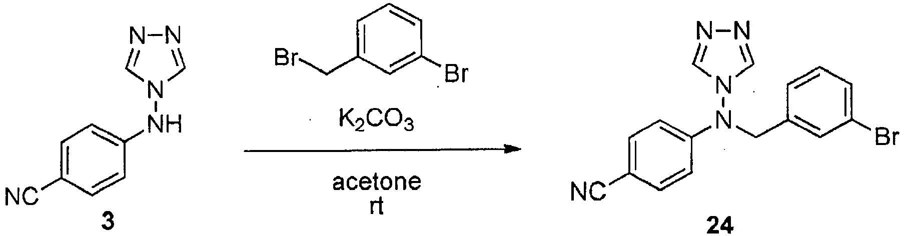

Under argon atmosphere, an acetone (15 mL) solution of a mixture of Compound (3) (300 mg, 1.62 mmol), 3-bromobenzyl bromide (485 mg, 1.94 mmol), and potassium carbonate (448 mg, 3.24 mmol) was stirred at room temperature for 5 hours. Water (50 mL) was added to the mixture, and the mixture was extracted using CH₂Cl₂ (50 mL × 3). All the organic phases were mixed, sequentially rinsed with water (50 mL × 1) and a saline solution (50 mL × 1), dried (Na₂SO₄), filtered, and concentrated under reduced pressure. The residue was purified by flash column chromatography (20 g of silica gel, CH₂Cl₂/CH₃OH = 40/1) to obtain Compound (24) (colorless solid, 511 mg, 89.3%). Recrystallization of the Compound (24) with ethyl acetate (13 mL) and n-hexane (7 mL) resulted in colorless plate crystal (380 mg, 66.4%).
TLC R_{f} = 0.53 (CH₂Cl₂/CH₃OH = 9/1)
¹H NMR (300 MHz, CDCl₃) δ 4.88 (s, 2H, benzylic CH₂), 6.62-6.70 (AA'BB', 2H, aromatic), 7.14 (d, 1H, J = 7.9 Hz,aromatic), 7.23 (dd, 1H, J = 7.9, 7.9 Hz, aromatic), 7.43 (s, 1H, aromatic), 7.51 (d, 1H, J = 7.9 Hz, aromatic), 7.57-7.65 (AA'BB', 2H, aromatic), 8.18 (s, 2H, triazole)
¹³C NMR (67.8 MHz, DMSO-d₆) δ 56.6, 103.0, 113.7 (2C), 119.0, 121.9, 127.4, 130.8, 131.0, 131.1, 133.9 (2C), 137.6, 143.3 (2C), 151.3
IR (KBr, cm⁻¹) 546, 615, 664, 700, 735, 787, 826, 997, 1067, 1138, 1179, 1206, 1273, 1298, 1331, 1395, 1429, 1474, 1508, 1572, 1605, 2222, 3117
Anal. Calcd. for C₁₆H₁₂N₅Br: C, 54.25; H, 3.41; N, 19.77. Found: C, 54.25; H, 3.51; N, 19.84.

### (IX) Synthesis of Compound (25) (4-{(4-Fluoromethylbenzyl)(4H-1,2,4-triazol-4-yl)amino}benzonitrile)

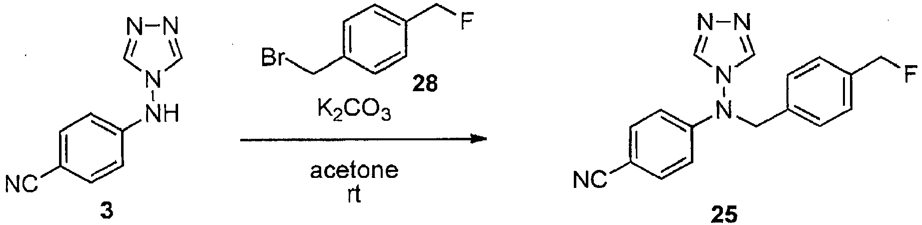

Compound (28) was synthesized according to the scheme below.

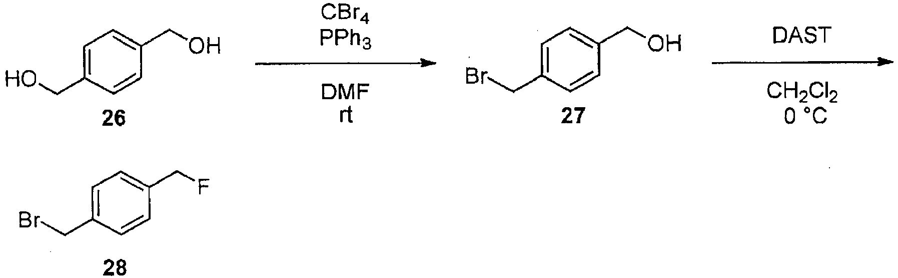

### (i) Synthesis of Compound (27) (4-Bromomethylbenzyl alcohol) (see Non-patent Literature 16 (Vassiliou, S,; Xeilari, M.; Yiotakis, A,; Grembecka, J.; Pawelczak, M.; Kafarskib, P.; Muchab, A., Bioorg. Med. Chem. 2007, 15, 3187-3200))

Under argon atmosphere, triphenylphosphine (14.2 g, 54.3 mmol) was added to a DMF (50 mL) solution of Compound (26) (5.00 g, 36.2 mmol) and carbon tetrabromide (18.0 g, 54.3 mmol) at 0 °C, and the mixture was heated up to room temperature. The mixture was stirred for 1 hour, and then water (300 mL) was added to the mixture, and the mixture was extracted with ethyl acetate (100 mL × 3). All the organic phases were mixed, sequentially rinsed with water (100 mL × 3) and a saline solution (100 mL × 1), dried (Na₂SO₄), filtered, and concentrated under reduced pressure.

The residue was purified by flash column chromatography (220 g of silica gel, n-hexane/EtOAc = 4/1) to obtain Compound (27) (colorless solid, 3.28 g, 45.1%).
TLC R_{f} = 0.25 (n-hexane/EtOAc = 3/1)
¹H NMR (270 MHz, CDCl₃) δ 1.78 (br s, 1H, OH), 4.50 (s, 2H, benzylic CH₂), 4.69 (s, 2H, benzylic CH₂), 7.30-7.36 (AA'BB', 2H, aromatic), 7.37-7.45 (AA'BB', 2H, aromatic).

### (ii) Synthesis of Compound (28) (4-Fluoromethylbenzyl bromide) (see Non-patent Literature 17 (Ichikawa, J.; Sugimoto, K.; Sonoda, T.; Kobayashi, H., Chem. Lett. 1987, 10, 1985-1988))

Under argon atmosphere, (diethylamino)sulfur trifluoride (DAST) (660 µL, 5.04 mmol) was added to a CH₂Cl₂ (10 mL) solution of Compound (27) (500 mg, 2.49 mmol) at 0 °C, and the mixture was stirred at 0 °C for 1 hour. NaHCO₃-saturated aqueous solution was added to the mixture, and the mixture was extracted with CH₂Cl₂ (50 mL × 3). All the organic phases were mixed, rinsed with water (50 mL × 3), dried (Na₂SO₄), filtered, and concentrated under reduced pressure.

The residue was purified by flash column chromatography (30 g of silica gel, n-hexane/Et₂O = 50/1) to obtain Compound (28) (yellowish white solid, 352 mg, 69.7%).
TLC R_{f} = 0.52 (n-hexane/EtOAc = 6/1)
¹H NMR (300 MHz, CDCl₃) δ 4.50 (s, 2H, benzylic CH₂), 5.38 (d, 2H, J_{H-F} = 47.6 Hz, benzylic CH₂), 7.33-7.40 (AA'BB', 2H, aromatic), 7.41-7.47 (AA'BB', 2H, aromatic).

### (iii) Synthesis of Compound (25) (4-{(4-Fluoromethylbenzyl)(4H-1,2,4-triazol-4-yl)amino}benzonitrile)

Under argon atmosphere, an acetone (10 mL) solution of a mixture of Compound (3) (191 mg, 1.03 mmol), Compound (28) (251 mg, 1.24 mmol), and potassium carbonate (285 mg, 2.06 mmol) was stirred at room temperature for 4 hours. Water (50 mL) was added to the mixture, and the mixture was extracted using CH₂Cl₂ (30 mL × 3). All the organic phases were mixed, rinsed with water (30 mL × 1), dried (Na₂SO₄), filtered, and concentrated under reduced pressure.

The residue was purified by flash column chromatography (30 g of silica gel, CH₂Cl₂/CH₃OH = 50/1) to obtain Compound (25) (pale yellow solid, 264 mg, 83.3%).
TLC R_{f} = 0.54 (CH₂Cl₂/CH₃OH = 9/1)
Mp 158-159 °C
¹H NMR (300 MHz, CDCl₃) δ 4.92 (s, 2H, benzylic CH₂), 5.38 (d, 2H, J_{H-F} = 47.4 Hz, benzylic CH₂), 6.63-6.73 (AA'BB', 2H, aromatic), 7.20-7.30 (AA'BB', 2H, aromatic), 7.33-7.43 (AA'BB', 2H, aromatic), 7.55-7.65 (AA'BB', 2H, aromatic), 8.13 (s, 2H, triazole)
¹³C NMR (67.8 MHz, DMSO-d₆) δ 57.0, 83.8 (d, ¹J_{C-F} = 162.0 Hz), 102.9, 113.7 (2C), 119.0, 128.1 (2C, d, ³J_{C-F} = 5.6 Hz), 128.6 (2C), 133.9 (2C), 135.3 (d, ⁵J_{C-F} = 3.3 Hz), 136.0 (d, ²J_{C-F} = 16.2 Hz), 143.3 (2C), 151.5
¹⁹F NMR (282 MHz,CDCl₃) δ -47.9 (t, J_{F-H} = 47.4 Hz)
IR (KBr, cm⁻¹) 546, 669, 735, 826, 951, 1001, 1067, 1179, 1219, 1298, 1333, 1379, 1423, 1460, 1510, 1603, 2222, 3117
Anal. Calcd. for C₁₇H₁₄N₅F: C, 66.44; H, 4.59; N, 22.79. Found: C, 66.51; H, 4.79; N, 22.69.

### (X) Synthesis of Compound 33 (4-{(4-[¹¹C]Methylbenzyl)(4H-1,2,4-triazol-4-yl)amino}nitrobenzene)

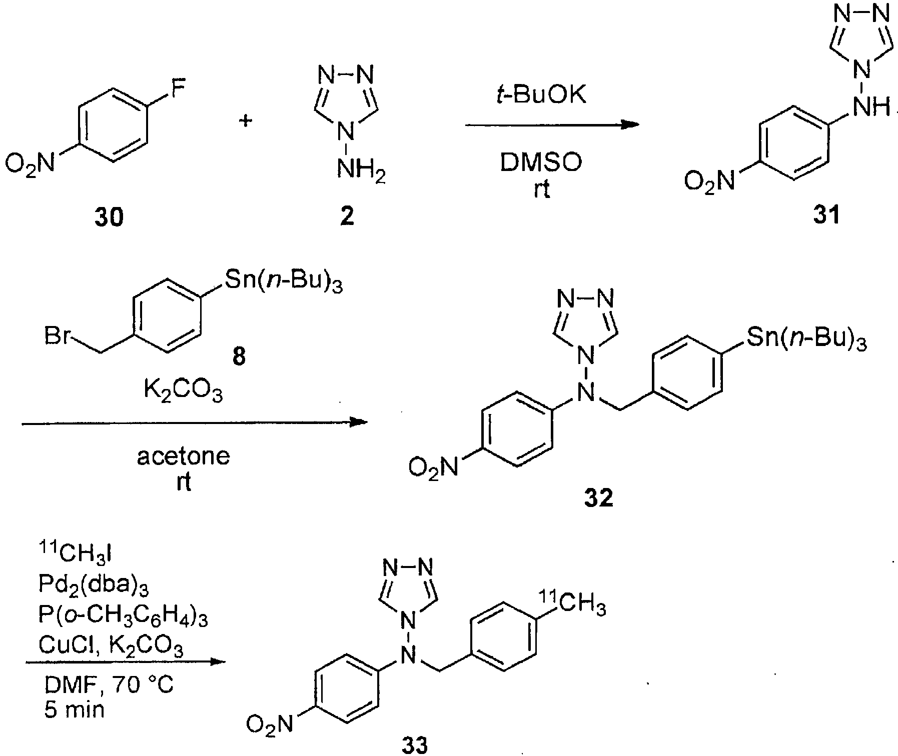

### (i) Synthesis of Compound 32 (4-[(4H-1,2,4-Triazol-4-yl){4-(tributylstannyl)benzyl}amino]nitrobenzene)

Under argon atmosphere, an acetone (20 mL) solution of a mixture of Compound (31) (449 mg, 2.19 mmol), Compound (8) (1.21 g, 2.63 mmol), and potassium carbonate (605 mg, 4.38 mmol) was stirred at room temperature for 18 hours. Water (100 mL) was added to the mixture, and the mixture was extracted using ethyl acetate (80 mL × 3).

All the organic phases were mixed, sequentially rinsed with water (50 mL × 1) and a saline solution (50 mL × 1), dried (Na₂SO₄), filtered, and concentrated under reduced pressure.

The residue was purified by flash column chromatography (60 g of silica gel, n-hexane/EtOAc = 1/1) to obtain Compound (32) (pale brown solid, 718 mg, 56.1%). Recrystallization of Compound (32) with ethyl acetate (3 mL) and n-hexane (30 mL) resulted in pale yellow plate crystal (534 mg, 41.8%).
TLC R_{f} = 0.43 (n-hexane/EtOAc = 1/1)
Mp 112-113 °C
¹H NMR (300 MHz, CDCl₃) δ 0.88 (t, 9H, J = 7.2 Hz, 3CH₃), 0.94-1.19 (m, 6H, 3CH₂), 1.24-1.40 (m, 6H, 3CH₂), 1.41-1.63 (m, 6H, 3CH₂), 4.94 (s, 2H, benzylic CH₂), 6.61-6.72 (AA'BB', 2H, aromatic), 7.11-7.21 (AA'BB', 2H, aromatic), 7.36-7.55 (AA'BB', 2H, ³J(^{119/117}Sn-¹H) = 36.0 Hz, aromatic), 8.14 (s, 2H, triazole), 8.16-8.22 (AA'BB', 2H, aromatic)
¹³C NMR (67.8 MHz,CDCl₃) δ 9.6 (3C, ¹J(¹¹⁹Sn-¹³C) = 341.9 Hz, ¹J(¹¹⁷Sn-¹³C) = 327.4 Hz), 13.6 (3C), 27.2 (3C, 3J(^{119/117}n-¹³C) = 56.5 Hz), 28.9 (3C, ²J(^{119/117}Sn-¹³C) = 20.1 Hz), 58.3, 112.3 (2C), 126.0 (2C), 127.2 (2C, ³J(^{119/117}Sn-¹³C) = 39.1 Hz), 132.8, 137.3 (2C, ²J(^{119/117}Sn-¹³C) = 30.8 Hz), 141.7, 142.6 (2C), 143.8, 151.9
IR (KBr, cm⁻¹) 596, 617, 637, 665, 691, 750, 837, 860, 997, 1016, 1065, 1123, 1132, 1196, 1221, 1263, 1339, 1375, 1395, 1460, 1501, 1595, 2851, 2924, 3117
Anal. Calcd. for C₂₇H₃₉N₅O₂Sn: C, 55.50; H, 6.73; N, 11.99. Found: C, 55.48; H, 6.36; N, 11.95.

### (ii) Synthesis of Compound 33 (4-{(4-[¹¹C]Methylbenzyl)(4H-1,2,4-triazol-4-yl)amino}nitrobenzene)

Using a cyclotron (product name; Sumitomo CYPRIS HM-12S cyclotron, manufactured by Sumitomo Heavy Industries, Ltd.), [¹¹C]carbon dioxide was synthesized by ¹⁴N(p,α)¹¹C reaction, and then lithium aluminum hydride and hydriodic acid were added in this order by a labeling synthesis device (RIKEN original automated radiolabeling system) so that the resultant was converted into [¹¹C]methyl iodide. The resulting [¹¹C]methyl iodide was used in a high-speed C-[¹¹C]methylation reaction using palladium(0) catalyst shown below.

[¹¹C]Methyl iodide was transferred by He gas flow (30 mL/min) to a DMF (0.3 mL) solution of Pd₂(dba)₃ (2.0 mg, 1.8 µmol) and P(o-tolyl)₃(3.0 mg, 9.5 µmol) in the reaction vessel (A) at room temperature. The resulting mixture was transferred to the reaction vessel (B) containing the Compound (32) (3.0 mg, 5.1 µmol), CuCl (2.0 mg, 20 µmol), and K₂CO₃ (2.4 mg, 18 µmol). The inside of the reaction vessel (A) was rinsed with DMF (0.5 mL) and the resulting solution was transferred to the reaction vessel (B).

The resulting mixture was heated at 70 °C for 5 min. The salt and palladium residues in the reaction mixture were removed by solid-phase extraction and rinsing with 1 mL of CH₃CN:30 mM CH₃COONH₄ (40:60) solution.

The combined eluate was injected into a preparative HPLC equipped with a γ detector (mobile phase, CH₃CN:30 mM CH₃COONH₄ (40:60); column, Nacalai COSMOSIL AR-II C18, 10 mm × 250 mm; guard column, Nacalai COSMOSIL Cholester or Sumika SUMIPAK Filter PG-ODS; flow rate, 6 mL/min; UV detection wavelength, 225 nm; retention time of Compound (33), 12 min). The fraction of interest was put in a flask and the organic solvent was removed under reduced pressure.

The ¹¹C-labeled compound of interest was dissolved in a mixture of polysorbate 80 (0.05 mL), propylene glycol (0.3 mL) and a physiological saline (4 mL).

The total time for all synthetic processes including HPLC purification and preparation of radioactive drug for intravenous administration was 38 min. The Compound (33) prepared for administration exhibited radioactivity of 0.5-2.6 GBq and specific radioactivity of 20-44 GBq/µmol.

The Compound (33) was identified by injecting, together with Compound (34) which is a non-radiolabeled authentic sample, into an analytical HPLC equipped with a γ detector (mobile phase, CH₃CN:30 mM CH₃COONH₄ (40:60); column, Nacalai COSMOSIL AR-II C18, 4.6 mm × 100 mm; flow rate, 1 mL/min; UV detection wavelength, 254 nm; retention time of Compound (33), 6.1 min). The chemical purity and the radiochemical purity were 95 % or higher.

### (XI) Synthesis of Compound (34) (4- {(4-Methylbenzyl)(4H-1,2,4-triazol-4-yl)amino}nitrobenzene) (see Non-patent Literature 3)

As a control for the Compound (33), Compound (34) which is not radiolabeled was synthesized according to the scheme below.

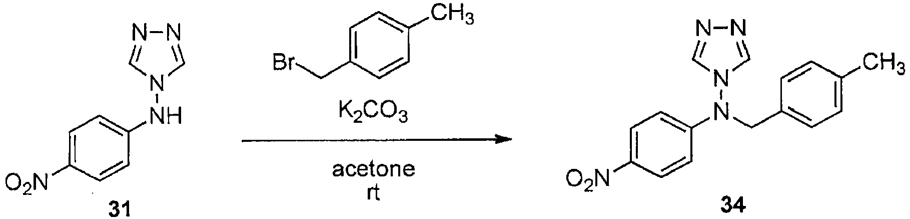

Under argon atmosphere, an acetone (15 mL) solution of a mixture of Compound (31) (500 mg, 2.44 mmol), 4-methylbenzyl bromide (542 mg, 2.93 mmol), and potassium carbonate (674 mg, 4.88 mmol) was stirred at room temperature for 22 hours. Water (100 mL) was added to the mixture, and the mixture was extracted using ethyl acetate (100 mL × 3). All the organic phases were mixed, sequentially rinsed with water (50 mL × 1) and a saline solution (50 mL × 1), dried (Na₂SO₄), filtered, and concentrated under reduced pressure.

The residue was purified by flash column chromatography (60 g of silica gel, n-hexane/EtOAc = 1/2) to obtain Compound (34) (pale brown solid, 340 mg, 45.1%). Recrystallization of the Compound (34) with ethyl acetate (60 mL) and n-hexane (30 mL) resulted in pale yellow plate crystal (254 mg, 33.7%).
TLC R_{f} = 0.48 (n-hexane/EtOAc = 1/4)
¹H NMR (300 MHz, CDCl₃) δ 2.35 (s, 3H, CH₃), 4.91 (s, 2H, benzylic CH₂), 6.63-6.73 (AA'BB', 2H, aromatic), 7.05-7.12 (AA'BB', 2H, aromatic), 7.13-7.20 (AA'BB', 2H, aromatic), 8.10 (s, 2H, triazole), 8.15-8.20 (AA'BB', 2H, aromatic)
¹³C NMR (75.5 MHz, DMSO-d₆) δ 20.7, 57.0, 113.0 (2C), 125.8 (2C), 128.5 (2C), 129.3 (2C), 131.4, 137.4, 140.6, 143.3 (2C), 153.1
IR (KBr, cm⁻¹) 665, 748, 818, 835, 851, 870, 1063, 1111, 1188, 1211, 1223, 1288, 1335, 1395, 1495, 1595, 3067, 3123
Anal. Calcd. for C₁₆H₁₅N₅O₂: C, 62.13; H, 4.89; N, 22.64. Found: C, 62.21; H, 4.88; N, 22.68.

### (XII) Synthesis of Compound 35 (4-{(4-Bromobenzyl)(4H-1,2,4-triazol-4-yl)amino}nitrobenzene)

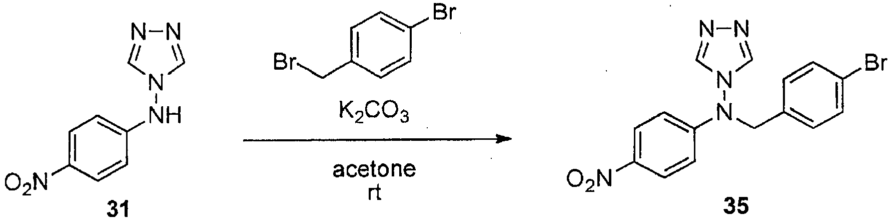

Under argon atmosphere, an acetone (15 mL) solution of a mixture of Compound (31) (500 mg, 2.44 mmol), 4-bromobenzyl bromide (732 mg, 2.93 mmol), and potassium carbonate (674 mg, 4.88 mmol) was stirred at room temperature for 22 hours. Water (100 mL) was added to the mixture, and the mixture was extracted using ethyl acetate (80 mL × 3).

All the organic phases were mixed, sequentially rinsed with water (50 mL × 1) and a saline solution (50 mL × 1), dried (Na₂SO₄), filtered, and concentrated under reduced pressure.

The residue was purified by flash column chromatography (60 g of silica gel, n-hexane/EtOAc = 1/2) to obtain Compound (35) (brown solid, 160 mg, 17.5%). Recrystallization of Compound (35) with ethyl acetate (30 mL) and n-hexane (10 mL) resulted in brown plate crystal (85.5 mg, 9.4%).
TLC R_{f} = 0.32 (n-hexane/EtOAc = 1/4)
¹H NMR (300 MHz, CDCl₃) δ 4.91 (s, 2H, benzylic CH₂), 6.63-6.71 (AA'BB', 2H, aromatic), 7.07-7.15 (AA'BB', 2H, aromatic), 7.48-7.55 (AA'BB', 2H, aromatic), 8.15 (s, 2H, triazole), 8.17-8.25 (AA'BB', 2H, aromatic)
¹³C NMR (67.8 MHz, DMSO-d₆) δ 56.6, 113.0 (2C), 121.4, 125.8 (2C), 130.6 (2C), 131.6 (2C), 134.0, 140.7, 143.3 (2C), 152.9
IR (KBr, cm⁻¹) 600, 665, 735, 750, 818, 849, 885, 908, 1015, 1063, 1111, 1223, 1277, 1341, 1503, 1591, 3125, 3429
Anal. Calcd. for C₁₅H₁₂N₅BrO₂: C, 48.15; H, 3.23; N, 18.72. Found: C, 48.24; H, 3.35; N, 18.56.

### (XIII) Synthesis of Compound (39) (4-{(4-[¹¹C]Methylbenzyl)(1H-1,2,4-triazol-1-yl)amino}benzonitrile)

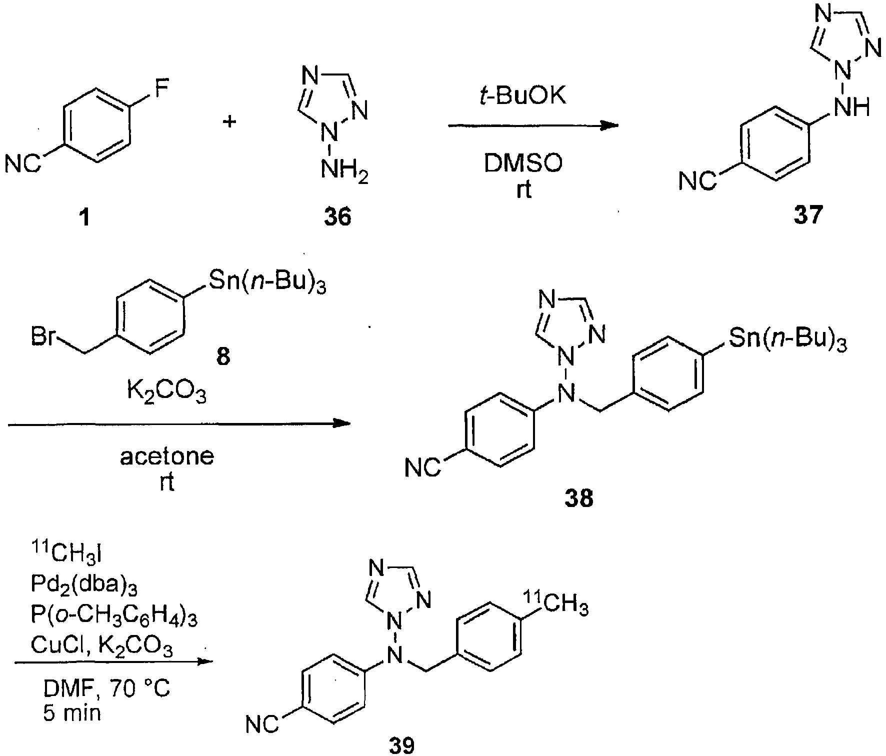

### (i) Synthesis of Compound 38 (4-[(1H-1,2,4-Triazo1-1-yl){4-(tributylstannyl)benzyl}amino]benzonitrile)

Under argon atmosphere, an acetone (15 mL) solution of a mixture of Compound (37) (300 mg, 1.62 mmol), Compound (8) (893 mg, 1.94 mmol), and potassium carbonate (448 mg, 3.24 mmol) was stirred at room temperature for 21 hours. Water (50 mL) was added to the mixture, and the mixture was extracted using CH₂Cl₂ (30 mL × 3). All the organic phases were mixed, rinsed with water (30 mL × 1), dried (Na₂SO₄), filtered, and concentrated under reduced pressure.

The residue was purified by flash column chromatography (60 g of silica gel, CH₂Cl₂ to CH₂Cl₂/EtOAc = 4/1) to obtain Compound (38) (colorless oily substance, 891 mg, 97.5%).
TLC R_{f} = 0.63 (CH₂Cl₂/EtOAc = 9/1)
¹H NMR (300 MHz, CDCl₃) δ 0.88 (t, 9H, J = 7.3 Hz, 3CH₃), 0.92-1.18 (m, 6H, 3CH₂), 1.23-1.39 (m, 6H, 3CH₂), 1.45-1.56 (m, 6H, 3CH₂), 4.89 (s, 2H, benzylic CH₂), 6.64-6.72 (AA'BB', 2H,aromatic), 7.16-7.23 (AA'BB', 2H, aromatic), 7.33-7.51 (AA'BB', 2H, ³J(^{119/117}Sn-¹H) = 36.9 Hz, aromatic), 7.52-7.61 (AA'BB', 2H, aromatic), 7.86 (s, 1H, triazole), 8.03 (s, 1H, triazole)
¹³C NMR (67.8 MHz, CDCl₃) δ 9.5 (3C, ¹J(¹¹⁹Sn-¹³C) = 341.4 Hz, ¹J(¹¹⁷Sn-¹³C) = 326.3 Hz), 13.6 (3C), 27.2 (3C, ³J(^{119/117}Sn-¹³C) = 55.8 Hz), 28:9 (3C, ²J(^{119/117}Sn-¹³C) = 20.6 Hz), 58.2, 105.2, 114.7 (2C), 118.7, 127.5 (2C, ³J(^{119/117}Sn-¹³C) = 39.6 Hz), 133.5, 133.6 (2C), 136.9 (2C, 2J(^{119/117}Sn-¹³C) = 30.2 Hz), 142.8, 143.9, 151.3, 151.4
IR (KBr, cm⁻¹) 669, 826, 986, 1130, 1279, 1273, 1375, 1454, 1508, 1605,2224,2851,2870,2924,2955
Anal. Calcd. for C₂₈H₃₉N₅Sn: C, 59.59; H, 6.97; N, 12.41. Found: C, 59.41; H, 6.86; N, 12.38.

### (ii) Synthesis of Compound 39 (4-{(4-[¹¹C]Methylbenzyl)(1H-1,2,4-triazol-1-yl)amino}benzonitrile)

Using a cyclotron (product name; Sumitomo CYPRIS HM-12S cyclotron, manufactured by Sumitomo Heavy Industries, Ltd.), [¹¹C]carbon dioxide was synthesized by ¹⁴N(p,α)¹¹C reaction, and then lithium aluminum hydride and hydriodic acid were added in this order by a labeling synthesis device (RIKEN original automated radiolabeling system) so that the resultant was converted into [¹¹C]methyl iodide. The resulting [¹¹C]methyl iodide was used in a high-speed C-[¹¹C]methylation reaction using palladium(0) catalyst shown below.

[¹¹C]Methyl iodide was transferred by He gas flow (30 mL/min) to a DMF (0.3 mL) solution of Pd₂(dba)₃ (2.7 mg, 2.4 µmol) and P(o-tolyl)₃ (3.0 mg, 9.5 µmol) in the reaction vessel (A) at room temperature. The resulting mixture was transferred to the reaction vessel (B) containing the Compound (38) (3.0 mg, 5.3 µmol), CuCl (2.0 mg, 20 µmol), and K₂CO₃ (2.4 mg, 18 µmol). The inside of the reaction vessel (A) was rinsed with DMF (0.5 mL) and the resulting solution was transferred to the reaction vessel (B).

The resulting mixture was heated at 70 °C for 5 min. The salt and palladium residues in the reaction mixture were removed by solid-phase extraction and rinsing with 1 mL of CH₃CN:30 mM CH₃COONH₄ (50:50) solution.

The combined eluate was injected into a preparative HPLC equipped with a γ detector (mobile phase, CH₃CN:30 mM CH₃COONH₄ (50:50); column, Nacalai COSMOSIL AR-II C18, 10 mm × 250 mm; guard column, Nacalai COSMOSIL Cholester or Sumika SUMIPAK Filter PG-ODS; flow rate, 5 mL/min; UV detection wavelength, 254 nm; retention time of Compound (39), 13.2 min). The fraction of interest was put in a flask and the organic solvent was removed under reduced pressure.

The ¹¹C-labeled compound of interest was dissolved in a mixture of polysorbate 80 (0.05 mL), propylene glycol (0.3 mL) and physiological saline (4 mL).

The total time for all synthetic processes including HPLC purification and preparation of radioactive drug for intravenous administration was 38 min. The Compound (39) prepared for administration exhibited radioactivity of 0.6-2.8 GBq and specific radioactivity of 50-116 GBq/µmol.

The Compound (39) was identified by injecting, together with Compound (38) which is a non-radiolabeled authentic sample, into an analytical HPLC equipped with a γ detector (mobile phase, CH₃CN:30 mM CH₃COONH₄ (50:50); column, Nacalai COSMOSIL AR-II C18, 4.6 mm × 100 mm; flow rate, 1 mL/min; UV detection wavelength, 254 nm; retention time of Compound (39), 4.5 min). The chemical purity and the radiochemical purity were 95% or higher.

### (XIV) Synthesis of Compound 40 (4-{(4-Methylbenzyl)(1H-1,2,4-triazol-1-yl)amino}benzonitrile) (see Non-patent Literature 4)

As a control for the Compound (39), Compound (40) which is not radiolabeled was synthesized according to the scheme below.

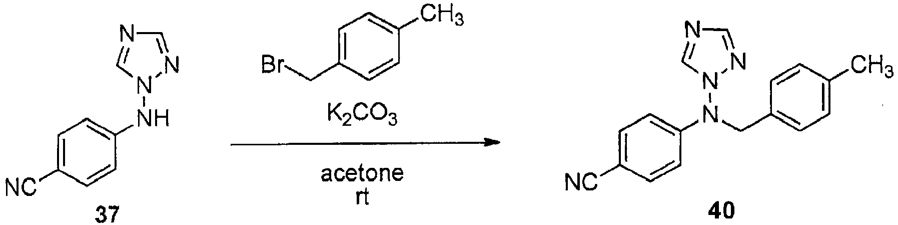

Under argon atmosphere, an acetone (15 mL) solution of a mixture of Compound (37) (300 mg, 1.62 mmol), 4-methylbenzyl bromide (359 mg, 1.94 mmol), and potassium carbonate (448 mg, 3.24 mmol) was stirred at room temperature for 20 hours. The mixture was concentrated under reduced pressure, and then water (50 mL) was added to the mixture, and the mixture was extracted using CH₂Cl₂ (30 mL × 3). All the organic phases were mixed, rinsed with water (30 mL × 1), dried (Na₂SO₄), filtered, and concentrated under reduced pressure.

The residue was purified by flash column chromatography (30 g of silica gel, CH₂Cl₂ to CH₂Cl₂/EtOAc = 4/1) to obtain Compound (40) (colorless solid, 448 mg, 95.6%). Recrystallization of the Compound (40) with ethyl acetate (8 mL) and n-hexane (32 mL) resulted in colorless plate crystal (395 mg, 84.3%).
TLC R_{f} = 0.46 (CH₂Cl₂/CH₃OH = 9/1)
¹H NMR (270 MHz, DMSO-d₆) δ 2.25 (s, 3H, CH₃), 4.96 (s, 2H, benzylic CH₂), 6.68-6.81 (AA'BB', 2H, aromatic), 7.04-7.16 (AA'BB', 2H, aromatic), 7.17-7.27 (AA'BB', 2H, aromatic), 7.66-7.81 (AA'BB', 2H, aromatic), 8.14 (s, 1H, triazole), 8.58 (s, 1H, triazole)
¹³C NMR (67.8 MHz, DMSO-d₆) δ 20.7, 56.7, 103.3, 114.6 (2C), 119.0, 128.4 (2C), 129.1 (2C), 131.7, 133.7 (2C), 137.2, 145.0, 151.0, 151.4
IR (KBr, cm⁻¹) 548, 617, 669, 719, 827, 943, 988, 1022, 1130, 1179, 1204, 1229, 1275, 1304, 1333, 1350, 1368, 1420, 1450, 1510, 1605, 2222, 2864, 2920,3049,3123
Anal. Calcd. for C₁₇H₁₅N₅: C, 70.57; H, 5.23; N, 24.21. Found: C, 70.76; H, 5.20; N, 24.29.

### (XV) Synthesis of Compound 41 (4-{(4-Bromobenzyl)(1H-1,2,4-triazol-1-yl)amino}benzonitrile) (see Non-patent Literature 4)

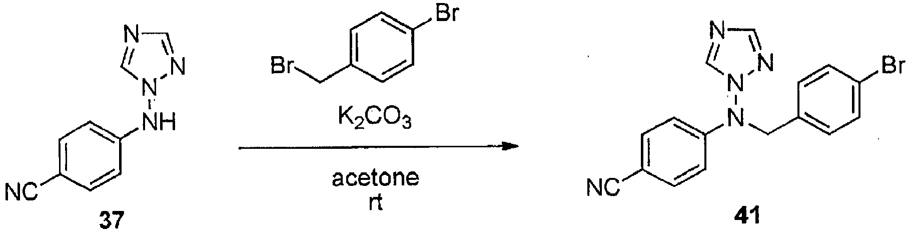

Under argon atmosphere, an acetone (15 mL) solution of a mixture of Compound (37) (300 mg, 1.62 mmol), 4-bromobenzyl bromide (485 mg, 1.94 mmol), and potassium carbonate (448 mg, 3.24 mmol) was stirred at room temperature for 20.5 hours. The mixture was concentrated under reduced pressure, and then water (50 mL) was added to the mixture, and the mixture was extracted using CH₂Cl₂ (30 mL × 3). All the organic phases were mixed, rinsed with water (50 mL × 1), dried (Na₂SO₄), filtered, and concentrated under reduced pressure.

The residue was purified by flash column chromatography (60 g of silica gel, CH₂Cl₂ to CH₂Cl₂/EtOAc = 4/1) to obtain Compound (41) (colorless solid, 575 mg, quant.). Recrystallization of the Compound (41) with ethyl acetate (10 mL) and n-hexane (30 mL) resulted in colorless plate crystal (458 mg, 79.8%).
TLC R_{f} = 0.48 (CH₂Cl₂/EtOAc = 9/1)
¹H NMR (300 MHz,CDCl₃) δ 4.87 (s, 2H, benzylic CH₂), 6.64-6.75 (AA'BB', 2H, aromatic), 7.09-7.22 (AA'BB', 2H, aromatic), 7.41-7.52 (AA'BB', 2H, aromatic), 7.53-7.65 (AA'BB', 2H, aromatic), 7.87 (s, 1H, triazole), 8.03 (s, 1H, triazole)
¹³C NMR (67.8 MHz,DMSO-d₆) δ 56.2, 103.5, 114.6 (2C), 119.0, 121.1, 130.7 (2C), 131.4 (2C), 133.8 (2C), 134.4, 145.0, 151.1, 151.2
IR (KBr, cm⁻¹) 509, 548, 615, 669, 735, 827, 943, 986, 1013, 1070, 1130, 1179, 1204, 1227, 1275, 1333, 1373, 1406, 1445, 1508, 1605, 1767, 2222, 3121
Anal. Calcd. for C₁₆H₁₂N₅Br: C, 54.25; H, 3.41; N, 19.77. Found: C, 54.28; H, 3.40; N, 19.96.

### [Evaluation of compound]

### (I) PET Measurement

(i) Male Rhesus macaques were subjected to PET measurement. Specifically, Rhesus macaques were put under sedation with ketalar (10 mg/kg), and intravenous catheters for RI administration were attached to their legs. Thereafter, the Rhesus macaques were anesthetized using propofol (10 mg/kg/h), and were immobilized in a PET scanner.
   In the PET measurement, after 30 minutes of transmission scanning, the Compound (10) (33 MBq/kg) was intravenously injected to the Rhesus macaques, and they were scanned for 90 min. After reconstructing images, binding potential (BP) images with cerebellum as a reference region were constructed. The binding potential used herein indicates a value obtained by dividing an association rate constant by a dissociation rate constant.
   For comparison, the same operation was carried out separately using [¹¹C]vorozole (see Non-patent Literatures 1 and 2 in the Background Art) instead of the Compound (10). These results are shown in Figs. 2-4.
   In Figs. 2 and 3, "coronal" indicates a forehead (coronal) cross-section and "transaxial" indicates a horizontal cross-section.
   Fig. 2 is a drawing showing binding potential (BP) images of amygdala in a case of using the Compound (10) of the present invention and in a case of a conventional [¹¹C]vorozole (VOR). Arrows in Fig. 2 indicate amygdala.
   As is obvious from Fig. 2, specific binding was observed in amygdala in the case of using the Compound (10) of the present invention as well as in the case of using a conventional [¹¹C]vorozole (VOR). Further, nonspecific binding of the labeled compound to cerebral cortex etc. was decreased in the case of using the Compound (10) of the present invention compared to the case of using a conventional [¹¹C]vorozole (VOR).
   Fig. 3 is a drawing showing binding potential (BP) images of nucleus accumbens in the case of using the Compound (10) of the present invention and in the case of using a conventional [¹¹C]vorozole (VOR). Arrows in Fig. 3 indicate nucleus accumbens.
   As is obvious from Fig. 3, similar behaviors were observed also in nucleus accumbens.
   Fig. 4 is a graph showing temporal changes in SUV (standardized uptake value) in PET measurement of cerebellum, amygdala, hypothalamus, and nucleus accumbens. (a) of Fig. 4 shows the case of using a conventional [¹¹C]vorozole (VOR), and (b) of Fig. 4 shows the case of using the Compound (10) of the present invention. SUV is a value obtained by dividing radioactivity density per 1 ml of tissue by radioactivity density per 1 g of body weight, i.e. $SUV = radioactivity density per 1 ml of tissue / radioactivity density per 1 g of body weight .$
   In Fig. 4, cerebellum, amygdala, hypothalamus, and nucleus accumbens are represented by "CRB", "Amy", "HT" and "Acb", respectively. As shown in Fig. 4, in the case of using a conventional [¹¹C]vorozole (VOR), SUV does not change or increases a little as time passes in the latter part of scanning. This indicates that there is a possibility that metabolite was taken into the brain again.
   On the other hand, in the case of using the Compound (10) of the present invention, SUV decreases as time passes in the latter part of scanning. This indicates that there is little possibility for the metabolite to be taken into the brain again. In view of the above, it is considered that the use of the Compound (10) of the present invention allows quantifying aromatase in the brain with higher accuracy.
   In order to confirm reuptake of labeled metabolite, the Compound (10) was injected into tail veins of male rats anesthetized with isoflurane. 5, 10 and 20 min after the injection, bloods were sampled from aorta adbominalis and then the brains were taken out and homogenized. Acetonitrile was added to the bloods and homogenated brains and the resultants were subjected to centrifugation, and extracts were subjected to HPLC in order to measure labeled metabolites.
   Fig. 8 shows content of parent compound in blood. Fig. 9 shows content of labeled metabolite in the brain.
   Data for "Monkey" shown in Fig. 8 was obtained by sampling vein blood along with PET imaging, centrifuging the sampled vein blood, adding acetonitrile to the blood plasma and centrifuging the blood plasma, and subjecting the extract to HPLC to measure parent compound in the blood plasma.
   The result shows that in the case of VOR, the labeled metabolite was taken into the brain again, but in the case of the Compound (10) of the present invention, such reuptake was not observed, as shown in Fig. 9.
(ii) Compounds (22), (33), and (39)
   PET measurements were carried out in the same manner as above except that the Compounds (22), (33), and (39), respectively, were used instead of the Compound (10). These results are shown in Figs. 10-13.

As is obvious from Fig. 10, in the cases of using the Compounds (22), (33) and (39) of the present invention, specific binding was observed in amygdala similarly with the case of using the Compound (10).

Further, as shown in Figs. 11-13, similarly with the case of using the Compound (10), SUV decreases as time passes in the latter part of scanning, and so it is considered that there is little possibility of reuptake of the metabolite. Therefore, use of the Compounds (22), (33) and (39) of the present invention allows quantifying aromatase in the brain with higher accuracy, too.

Although the use of the Compounds (22), (33) and (39) of the present invention exhibited the same distributions as that exhibited by the use of the Compound (10), there was observed a difference in binding potential among them. The Compound (22) exhibited relatively low binding potential, whereas the Compound (39) exhibited relatively high binding potential.

As described above, the use of the present invention allows visualizing distribution of aromatase which is an enzyme for converting male steroids into female steroids, and quantifying aromatase accurately. The use of the present invention allows significant decrease in nonspecific binding compared with the use of a conventional PET probe, thereby allowing quantification of aromatase with higher accuracy.

### (II) Replacement test using Compound (10) of the present invention

5 min before administration of the Compound (10), non-radiolabeled vorozole (VOR) (100 µg/kg) was administered, and binding potential (BP) of Rhesus macaques were observed.

In Fig. 5, the upper row (total binding) shows binding potential images in a case where only the Compound (10) was administered, and the lower row (replaced binding) shows binding potential (BP) images in a case where the Compound (10) was administered 5 min after administration of non-radiolabeled VOR (100 µg/kg).

Also in Fig. 5, "coronal" indicates a forehead (coronal) cross-section and "transaxial" indicates a horizontal cross-section.

Fig. 6 shows a graph obtained by putting the results of the binding images in Fig. 5 and the results of hypothalamus (HT) and nucleus accumbens (Acb) in numerals, as well as the results of the similar tests for VOR.

As shown in Fig. 5, in the case of administering the Compound (10) 5 min after administration of non-radiolabeled VOR, intake of the Compound (10) into amygdala, which was observed in the case of administering only the Compound (10), was not observed. This seems to be because non-radiolabeled VOR blocked the binding of the Compound (10). Similar results were obtained in the cases of hypothalamus and nucleus accumbens.

Further, as shown in Fig. 6, the Compound (10) of the present invention exhibited a smaller amount of replaced binding than the case of VOR. It is considered that the replaced binding was nonspecific binding. Accordingly, it was confirmed that the Compound (10) of the present invention exhibited significantly decreased nonspecific binding compared with a conventional PET probe and thus allows quantification of aromatase with higher accuracy.

### (III) Binding test of Compound (10) of the present invention

The binding test of the Compound (10) was carried out in vitro.

Specifically, female rats were anesthetized with diethyl ether, and 0.01 M phosphate buffered saline (PBS, pH 7.4) was perfused via hearts. Ovaries of the rats were removed immediately and homogenized in 1 mL of a 0.32 M sucrose solution.

For a ligand saturation test, the homogenized tissues were incubated in two incubators at room temperature for 30 min while increasing density (0.03, 0.05, 0.1, 0.3, 1.0, and 2.0 nM) of the Compound (10). Nonspecific binding was obtained by adding 1 µM of non-radiolabeled VOR before adding the Compound (10) and incubating a sample with the resultant. After filtration, counting was performed by a γ counter, and the average of K_{D} was calculated by Scatchard plot analysis.

Fig. 7 shows one example of the result of the binding test. (a) of Fig. 7 shows the result of VOR in amygdala in rats, and (b) of Fig. 7 shows the result of testing the Compound (10).

It is confirmed from these results that a dissociation constant (K_{D}) indicative of binding potential is 0.30 nM in the case of the Compound (10) and 0.60 nM in the case of vorozole (VOR), indicating that the Compound (10) of the present invention has approximately twice higher affinity than VOR.

### Industrial Applicability

The compound of the present invention is preferably applicable as a molecular probe for PET in use for visualizing distribution of aromatase and quantifying aromatase.

## Claims

1. A compound, comprising
a structure represented by any of the following formulae:

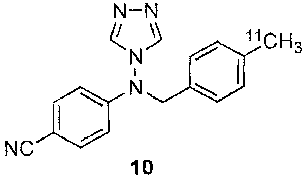

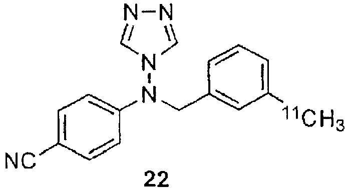

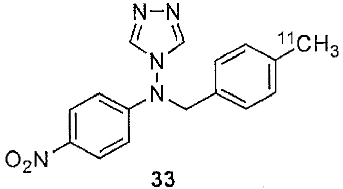

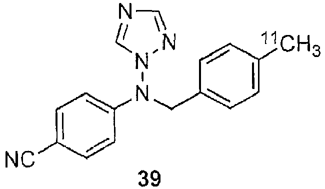

2. A composition for diagnosis, comprising a compound as set forth in claim 1.

3. The composition as set forth in claim 2, further comprising a compound having a structure represented by formula (1") 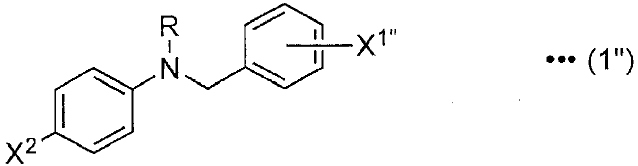 wherein X^{1"} is CH₃, X² is CN or NO₂ and R is any one of groups represented by formulae below, 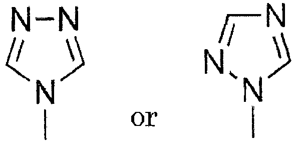 and wherein said compound of formula (1") has the same structure as that of the compound of claim 1, except that the compound of formula (1") is not radiolabelled.

4. The composition as set forth in claim 2 or 3 for use in diagnosis of a disease selected from the group consisting of diseases suspected to be related to aromatase or sex steroids, inflammatory diseases with induction of aromatase, mental disorders, menopausal syndrome, and chronic fatigue syndrome.

5. The composition as set forth in any one of claims 2-4 for use in diagnosis using PET.

## Patentansprüche

1. Verbindung, umfassend
eine Struktur, die durch eine der folgenden Formeln dargestellt ist:

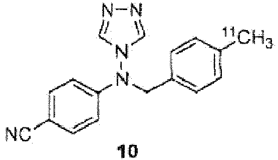

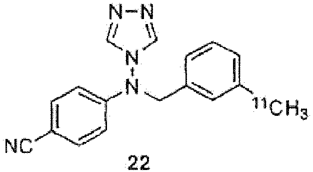

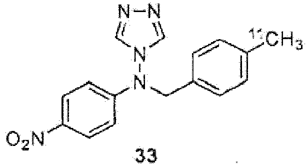

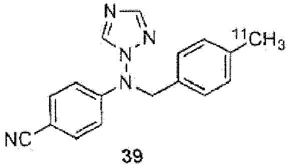

2. Zusammensetzung für die Diagnose, umfassend eine Verbindung, wie in Anspruch 1 aufgeführt,

3. Zusammensetzung, wie in Anspruch 2 aufgeführt, ferner eine Verbindung umfassend, die eine Struktur wie durch Formel (1") dargestellt, aufweist 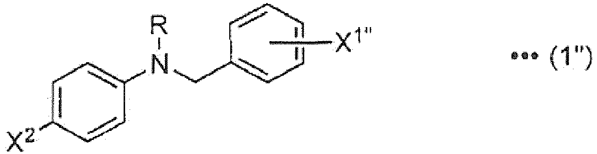 wobei X¹" CH₃ ist, X² CN oder NO₂ ist und R eine der Gruppen ist, die durch die Formeln unten dargestellt sind 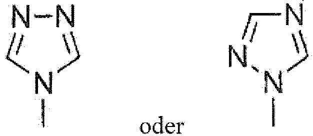 und wobei die Verbindung der Formel (1") dieselbe Struktur aufweist wie diejenige der Verbindung von Anspruch 1, mit der Ausnahme, dass die Verbindung der Formel (1") nicht radioaktiv markiert ist.

4. Zusammensetzung wie in Anspruch 2 oder 3 aufgeführt, zur Verwendung bei der Diagnose einer Krankheit, ausgewählt aus der Gruppe, bestehend aus Krankheiten, die mutmaßlich mit Aromatase oder Geschlechtssteroiden verbunden sind, Entzündungskrankheiten mit Induktion von Aromatase, psychischen Störungen, Menopausensyndrom und chronischem Erschöpfungssyndrom.

5. Zusammensetzung, wie in einem der Ansprüche 2 - 4 aufgeführt, zur Verwendung bei der Diagnose unter Anwendung von PET.

## Revendications

1. Composé comprenant une structure représentée par l'une quelconque des formules suivantes :

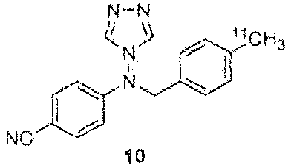

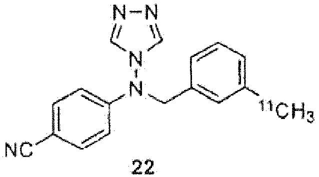

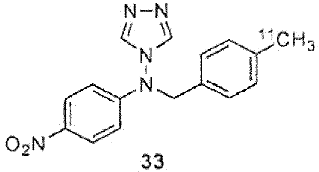

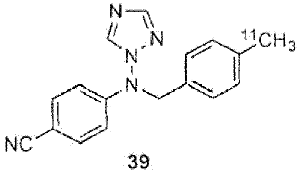

2. Composition de diagnostic, comprenant un composé selon la revendication 1.

3. Composition selon la revendication 2, comprenant en outre un composé répondant à une structure représentée par la formule (1") 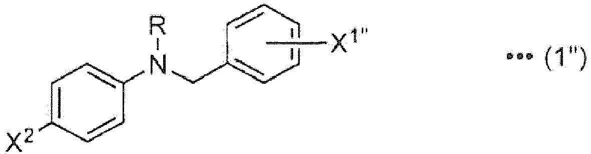 dans laquelle X^{1"} est CH₃, X² est CN ou NO₂, et R est l'un quelconque des groupes représentés par les formules ci-dessous 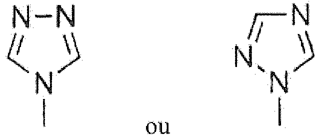 et dans laquelle ledit composé de formule (1") a la même structure que celle du composé selon la revendication 1, mis à part que le composé de formule (1") n'est pas radiomarqué.

4. Composition selon la revendication 2 ou 3, pour une utilisation dans un diagnostic d'une maladie sélectionnée dans le groupe constitué par les maladies soupçonnées d'être liées à l'aromatase ou aux stéroïdes sexuels, les maladies inflammatoires avec induction de l'aromatase, les troubles mentaux, le syndrome de ménopause et le syndrome de fatigue chronique.

5. Composition selon l'une quelconque des revendications 2 à 4, pour une utilisation dans un diagnostic utilisant une tomographie par émission de positions (TEP).
